# EUROPEAN PATENT APPLICATION

(11) **EP 3 311 820 A1**
(43) Date of publication of application: **25.04.2018**
(21) Application number: 17200433.5
(22) Date of filing: 26.02.2009
(51) Int. Cl.: A61K 31/56, A61K 9/00, A61K 31/40, A61K 45/06, A61P 11/00

(54) **METHOD AND SYSTEM FOR THE TREATMENT OF CHRONIC OBSTRUCTIVE PULMONARY DISEASE WITH NEBULIZED ANTICHOLINERGIC ADMINISTRATIONS**

(30) Priority: 26.02.2008 US 31639; 11.07.2008 US 80184
(62) Divisional of application: 09739326.8
(71) Applicant: Sunovion Respiratory Development Inc., Marlborough MA 01752-709 (US)
(72) Inventor: GERHART, William, La Mesa, CA California 91941 (US)
(74) Representative: Hutchins, Michael Richard

(57) **Abstract**

Inhalation solutions for administration of muscarinic antagonists for the treatment of breathing disorders, such as COPD, are provided.

## Description

This application claims priority under 35 U.S.C. § 119(e) from United States Provisional patent application 61/031,639, filed February 26, 2008, and from United States provisional patent application 61/080,184, filed July 11, 2008, each of which provisional is incorporated herein by reference in its entirety.

### BACKGROUND OF THE INVENTION

Chronic obstructive airway disease (COPD) is a pulmonary (lung) disease characterized by chronic obstruction of the airways. COPD encompasses emphysema and chronic bronchitis. Chronic bronchitis is diagnosed where a patient suffers from chronic cough, mucus production, or both, for at least three months in at least two successive years where other causes of chronic cough have been excluded. In chronic bronchitis, airway obstruction is caused by chronic and excessive secretion of abnormal airway mucus, inflammation, and bronchospasm. Often chronic bronchitis is exacerbated by frequent or chronic infection.

Emphysema involves the destruction of elastin in terminal bronchioles, which leads to remodeling, destruction and ultimate collapse of the airway walls. Patients with emphysema gradually lose the ability to exhale, causing a rise in blood waste gasses (such as carbon dioxide), a drop in blood oxygen, and a general degradation of patient stamina and overall health. A signal characteristic of emphysema is permanent loss of alveoli. Remodeling leads to permanent enlargement of the air spaces distal to the terminal bronchioles, and destruction of terminal bronchiole walls, though without fibrosis. Emphysema is progressive with a poor prognosis. Since there is no known method for repairing elastin or restoring the alveoli, therapy is generally palliative and persistent.

Patients suffering from COPD often suffer secondary pulmonary hypertension. Higher pulmonary blood pressure results in more work for the heart. As the heart enlarges in response to the higher work load, the distal extremities such as feet and ankles begin to swell with excess fluid. Eventually the heart tires, becoming unable to keep up with the additional work load, and ejection volume is decreased. As the heart's function degrades, fluid collects around the heart and lungs, leading eventually to a condition known as congestive heart failure.

Most patients suffering from COPD have both emphysema and chronic bronchitis. The standard of treatment for COPD includes maintenance and/or rescue dosing of antiinflammatory and/or bronchodilator aerosol drugs. While most patients respond to treatment with metered dose inhalers or dry powder inhalers, there is a subset of patients for whom such options are not well-suited. Older and sicker COPD patients, for example, often find it difficult to use metered dose inhalers and dry powder inhalers.

Dry powder inhalers are generally passive delivery devices, which patients actuate by forceful, controlled inhalation through the mouth. Metered dose inhalers, on the other hand, are in general active delivery devices, which create an atomized mist by forcing a drug solution or suspension through a nozzle under pressure. A patient activates the metered dose inhaler by pressing an actuator and simultaneously breathing in through the mouth in order to deposit the drug in the patient's lungs. Patients whose motor skills are impaired or not fully developed will often have trouble activating the device, coordinating their breathing, and generally using metered dose inhalers; and those patients whose inhalation capacity and control are impaired or not fully developed are often unable to properly operate dry powder inhalers. For these patients, nebulized delivery of drugs is an important delivery option, since the full drug can be administered to the patient with normal (tidal) breathing, albeit over multiple minutes.

There are two general categories of bronchodilators - muscarinic antagonists and beta 2-adrenergic agonists. Muscarinic antagonists are preferred and are recommended first-line therapy for maintenance treatment of COPD. Long-acting muscarinic antagonists (so-called LAMAs) are preferred to short-acting muscarinic antagonists, due to their superior efficacy and duration of effect. One LAMA that has been approved for use in COPD in the United States is tiotropium bromide powder for inhalation (Spiriva®, NDA No. 021395, Boehringer Ingelheim). Tiotriopium bromide is available commercially only as a dry powder, which is administered by a breath-activated inhaler. A similar mode of administration is disclosed by Bannister et al. (US 7,229,607) for administration of glycopyrronium bromide (glycopyrrolate) as a dry powder. The '607 patent distinguishes this methodology from administration of a solution formulation of glycopyrrolate, which is characterized as being unable to achieve effective treatment of COPD for longer than 12 hours. For example, Bannister et al. state: "Schroeckenstein et al., J. Allergy Clin. Immunol., 1988; 82(1): 115-119, discloses the use of glycopyrrolate in an aerosol formulation for treating asthma. A single administration of the metered-dose glycopyrrolate aerosol achieved bronchodilation over a 12 hour period." Additionally, Bannister et al. admit: "Skorodin, Arch Intern. Med, 1993; 153: 814 828, discloses the use of glycopyrrolate in an aerosol formulation for the treatment of asthma and COPD. It is stated that, in general, the quaternary ammonium anticholinergic compounds have a duration of action of 4 to 12 hours. A dose of between 0.2 to 1.0 mg of glycopyrrolate is recommended at 6 to 12 hour intervals." And the inventors of the '607 patent also state: "Walker et al., Chest, 1987; 91(1): 49-51, also discloses the effect of inhaled glycopyrrolate as an asthma treatment. Again, the duration of effective treatment is shown to be up to 12 hours, although up to 8 hours appears to be maximal."

Hansel et al. ("Glycopyrrolate causes prolonged bronchoprotection and bronchodilation in patients with asthma," 128 Chest 1974-1979 (2005)) claim to have demonstrated an improvement in bronchodilation and bronchoprotection in mild-to-moderate asthmatic patients for a period of up to 30 h with nebulized glycopyrronium bromide (glycopyrrolate). Single doses of glycopyrrolate (0.5, 1.0 and 2.0 mg/dose) were administered to mild-to-moderate asthmatic volunteers ages 18-60, who were then challenged with doubling increments of methacholine dose until a > 20% fall in FEV₁ was achieved. A log dose-response curve was constructed with these data by linear interpolation. No clear dose-response was observed for either bronchodilation or bronchoprotection. Although the authors claim to have demonstrated bronchodilation for a period of up to 30 h, the mean response in FEV₁ was clinically meaningful (>10% change from pre-dose levels) only at 2 hours post-dose and dropping to approximately 5% levels from 12 h through 30 h. This indicates that the bronchodilator response was short-lived and not sustainable beyond 12 hours. Thus, the authors failed to demonstrate prolonged bronchodilation with nebulized glycopyrrolate that was superior to ipratropium bromide. In the same study, the authors demonstrated clinically meaningful bronchoprotective effect of nebulized glycopyrrolate that was sustained up to 30 h at all dose levels. Although the bronchoprotective effect in response to a bronchial challenge test is considered a useful surrogate test for treatment of lung diseases with airway hyperresponsiveness, such as asthma, a positive bronchoprotective test is not considered a predictive tool and useful test in patients with COPD, because of the different disease pathology and mechanisms involved in COPD. As outlined in the literature and international guidelines, airway the hyperresponsiveness test is not considered a suitable test for use in COPD, therefore the data in asthma patients presented by Hansel et al. cannot be extrapolated for COPD. In any case, the authors seem to favor a breath-activated dry powder inhaler as a practical mode of administration of glycopyrrolate. Id. at 1978. Thus, at best, this study can be seen as being supportive of the dry powder inhalation methodology taught by Bannister et al. (US 7,229,607). Notably, none of the treated patients were identified as suffering from COPD. Nevertheless, Hansel et al. list as one of their conclusions that inhaled racemic glycopyrrolate would be superior to ipratropium bromide for treatment of stable COPD. Id. at 1974. Despite this prediction, no study has demonstrated effective treatment of COPD with nebulized glycopyrrolate for greater than 12 hours.

A sub-segment of the COPD population comprising the sickest and oldest patients require nebulizer delivery of their medicines because they are unable to satisfactorily operate a metered dose or dry powder inhaler. However, the treatment options for these patients are limited. Although two long-acting beta 2 agonist solution formulations are approved for nebulizer delivery and indicated for the maintenance of COPD symptoms, muscarinic antagonists are preferable for the treatment of COPD. Ipratropium bromide is the only muscarinic antagonist approved for nebulizer delivery in COPD (monotherapy or in combination with albuterol), however the frequent dosing and long nebulization times of this short-acting agent is inconvenient, leading to poor compliance and thus sub-optimal clinical outcomes. Longer acting aerosol drugs have been demonstrate to generally be more efficacious and result in better compliance compared to shorter acting drugs.

There is thus a need for additional therapeutic options for the treatment of COPD. There is a need for therapeutic options that offer greater convenience and better efficacy, especially for the sub-population of COPD patients who require nebulizer delivery. In particular there is a need for a nebulized muscarinic antagonist that provides more than 12 hours, and preferably at least 24 hours of therapeutic benefit to COPD patients. Heretofore, no method, device or system has been suggested that satisfies these needs.

### SUMMARY OF THE INVENTION

The foregoing and further needs are satisfied by embodiments of the present invention. Some embodiments provide long-acting treatment of one or more symptoms of COPD. Embodiments described herein provide methods, devices and systems that permit relief of one or more symptoms of COPD for a period of at least about 18 hr, preferably at least about 20 to 24 hours, with nebulized administration of an antimuscarinic agent, such as glycopyrronium bromide (glycopyrrolate). In particular embodiments set forth herein, there are provided methods, devices and systems for administration of an antimuscarinic agent, such as glycopyrrolate, via a high efficiency nebulizer. High efficiency nebulizers provide shorter treatment times and superior lung deposition as compared to conventional nebulizers. High efficiency nebulizers create smaller particle sizes with tighter particle size distributions, which results in more drug depositing in the lungs, and less drug depositing in the oropharyngeal pathway. This is particularly advantageous for delivering a muscarinic antagonist that has, as a dose-limiting side effect, dry mouth, which can limit the amount of drug that can be delivered to, and tolerated by, the patient, and thereby prevent achievement of maximal duration of therapeutic benefit. Also, the smaller particles tend to penetrate farther into the lungs, thereby enhancing distribution of the drug throughout the surface of the lungs and targeting a greater proportion of muscarinic receptors that are involved in the pathogenesis of one or more symptoms of COPD. Without wishing to be bound by theory, the inventors believe that the enhanced deposition, distribution or both of a long-acting muscarinic antagonist with a high efficiency nebulizer (as opposed to a conventional nebulizer) enhances outcomes in the treatment of COPD. In an exemplary embodiment, a nebulizer capable of delivering droplets having a median particle diameter of less than about 5 µm (especially less than about 4.5 µm) and a geometric standard deviation (GSD) of less than about 2.0,.1 (especially less than about 1.8) will provide more efficacious and better-tolerated treatment of COPD with an antimuscarinic agent as compared to a conventional nebulizer. Some embodiments provide a unit dosage form adapted or adaptable for administering a nominal dose of a muscarinic antagonist, such as glycopyrrolate, with a high efficiency nebulizer for treatment of COPD. Some embodiments provide a device comprising (1) a combination of a unit dosage form adapted or adaptable for administering a nominal dose of a muscarinic antagonist, such as glycopyrrolate, with a high efficiency nebulizer for treatment of COPD; and (2) a high efficiency nebulizer.

Furthermore, previous published reports of delivery of a glycopyrrolate solution formulation by nebulizer were at concentrations of no more than 0.2 mg/ml and contained a preservative, benzyl alcohol, that is a known lung irritant. Without wishing to be bound by theory, it is believed that a higher concentration of glycopyrrolate at the muscarinic receptor level and a more selective targeting of the muscarinic receptors (higher quantity of receptor binding) in the airways will contribute to a faster onset and/or greater magnitude of therapeutic effect and/or a greater duration of therapeutic effect. Additionally, eliminating the preservative enables higher or more concentrated doses of glycopyrrolate to be delivered in a better-tolerated manner.

Some embodiments described herein provide a method of treating a patient having chronic obstructive pulmonary disease (COPD), comprising administering to the patient, with a high efficiency nebulizer, a nominal dose of a composition comprising a muscarinic antagonist that provides the patient with a therapeutic effect for at least about 24 hours. Some embodiments comprise administering said nominal dose with the high efficiency nebulizer produces in the patient therapeutically acceptable side effects. In some embodiments, the method comprises administering said nominal dose of the composition with the high efficiency nebulizer provides to the patient reduced side effects compared to administering the same nominal dose with a conventional nebulizer. In some embodiments, the method comprises administering said nominal dose of the composition with the high efficiency nebulizer produces a calculated respirable dose of the muscarinic antagonist, whereby the patient experiences reduced side effects compared to administering a nominal dose that is calculated to achieve the same respirable dose with a conventional nebulizer. In some embodiments, the method comprises administering said nominal dose of the composition with the high efficiency nebulizer achieves a deposited lung dose of the muscarinic antagonist, whereby the patient experiences reduced side effects compared to administering a nominal dose that achieves substantially the same deposited lung dose with a conventional nebulizer. In some embodiments, the composition comprising the muscarinic antagonist is a concentrated, preservative-free, pH-adjusted solution formulation of the muscarinic antagonist. In some embodiments, the concentration of the muscarinic antagonist is greater than about 0.25 mg/mL, greater than about 0.5 mg/mL or greater than about 1 mg/mL. In some embodiments, the composition has a pH of 3 to 5. In some embodiments, the formulation is room temperature stable for at least 2 years. In some embodiments, the composition comprising the muscarinic antagonist contains about 50 µg to about 1000 µg of glycopyrrolate as the muscarinic antagonist. In some embodiments, the composition has a volume of about 1.0 mL or less, or optimally 0.5 mL or less. In some embodiments, the composition is administered in about 3 minutes or less. In some embodiments, the solution has a stabilizing excipient. In some embodiments, the stabilizing excipient is ethylenediaminetetraacetic acid (EDTA) or a pharmaceutically acceptable salt thereof. In some embodiments, the composition further comprises an excipient to mitigate side effects, for example dry mouth. In some embodiments, the excipient comprises citric acid or a pharmaceutically acceptable salt thereof. In some embodiments, the muscarinic antagonist is a long-acting muscarinic antagonist. In some embodiments, the nominal dose of the composition comprising the muscarinic antagonist contains about 50 µg to about 1000 µg of glycopyrrolate as the muscarinic antagonist. In some embodiments, the nominal dose of the composition comprising the muscarinic antagonist contains about 50 µg to about 500 µg of glycopyrrolate as the muscarinic antagonist. In some embodiments, the composition comprising the muscarinic antagonist contains about 50 µg to about 300 µg of glycopyrrolate as the muscarinic antagonist. In some embodiments, the composition comprising the muscarinic antagonist contains about 50 µg to about 150 µg of glycopyrrolate as the muscarinic antagonist. In some embodiments, the high efficiency nebulizer emits droplets having a Mass Median Aerodynamic Diameter (MMAD) of less than about 4.5 µm and a geometric standard deviation (GSD) of less than about 2.0,.0, an MMAD of less than 4.0 µm and a GSD less than 1.8, or optimally an MMAD less than 3.6 and a GSD less than 1.6. In some embodiments, the therapeutic effect comprises an improvement of FEV₁ above baseline of at least about 10% at 24 hours after the composition is administered with the high efficiency nebulizer. In some embodiments, the therapeutic effect comprises an improvement of FEV₁ above baseline of at least about 100 mL at 24 hours after the composition is administered with the high efficiency nebulizer. In some embodiments, the composition further comprises a beta 2-adrenoreceptor agonist, a corticosteroid, or both.

Some embodiments described herein provide a method of treating a patient having chronic obstructive pulmonary disease (COPD), comprising administering to the patient, with a high efficiency nebulizer, a nominal dose of a composition comprising a muscarinic antagonist, wherein administering said nominal dose with said high efficiency nebulizer provides to the patient: (1) an increased magnitude and/or duration of therapeutic effect; and (2) reduced or acceptable side effects, compared to administering the same nominal dose of the muscarinic antagonist with a conventional nebulizer. In some embodiments, the method comprises administering said nominal dose with the high efficiency nebulizer produces in the patient therapeutically acceptable side effects. In some embodiments, the method comprises administering said nominal dose of the composition with the high efficiency nebulizer provides to the patient reduced side effects compared to administering the same nominal dose with a conventional nebulizer. In some embodiments, the method comprises administering said nominal dose of the composition with the high efficiency nebulizer produces a calculated respirable dose of the muscarinic antagonist, whereby the patient experiences reduced side effects compared to administering a nominal dose that is calculated to achieve the same respirable dose with a conventional nebulizer. In some embodiments, the method comprises administering said nominal dose of the composition with the high efficiency nebulizer achieves a deposited lung dose of the muscarinic antagonist, whereby the patient experiences reduced side effects compared to administering a nominal dose that achieves substantially the same deposited lung dose with a conventional nebulizer. In some embodiments, the composition comprising the muscarinic antagonist is a concentrated, preservative-free, pH-adjusted solution formulation of the muscarinic antagonist. In some embodiments, the concentration of the muscarinic antagonist is greater than about 0.25 mg/mL, greater than about 0.5 mg/mL or greater than about 1 mg/mL, greater than about 0.5 mg/mL or greater than about 1 mg/mL. In some embodiments, the composition has a pH of 3 to 5. In some embodiments, the formulation is room temperature stable for at least 2 years. In some embodiments, the composition comprising the muscarinic antagonist contains about 50 µg to about 1000 µg of glycopyrrolate as the muscarinic antagonist. In some embodiments, the composition has a volume of about 1.0 mL or less, or optimally 0.5 mL or less. In some embodiments, the composition is administered in about 3 minutes or less. In some embodiments, the solution has a stabilizing excipient. In some embodiments, the stabilizing excipient is ethylenediaminetetraacetic acid (EDTA) or a pharmaceutically acceptable salt thereof. In some embodiments, the composition further comprises an excipient to mitigate side effects, for example dry mouth. In some embodiments, the excipient comprises citric acid or a pharmaceutically acceptable salt thereof. In some embodiments, the muscarinic antagonist is a long-acting muscarinic antagonist. In some embodiments, the nominal dose of the composition comprising the muscarinic antagonist contains about 50 µg to about 1000 µg of glycopyrrolate as the muscarinic antagonist. In some embodiments, the nominal dose of the composition comprising the muscarinic antagonist contains about 50 µg to about 500 µg of glycopyrrolate as the muscarinic antagonist. In some embodiments, the composition comprising the muscarinic antagonist contains about 50 µg to about 300 µg of glycopyrrolate as the muscarinic antagonist. In some embodiments, the composition comprising the muscarinic antagonist contains about 50 µg to about 150 µg of glycopyrrolate as the muscarinic antagonist. In some embodiments, the high efficiency nebulizer emits droplets having a Mass Median Aerodynamic Diameter (MMAD) of less than about 4.5 µm and a geometric standard deviation (GSD) of less than about 2.0, an MMAD of less than 4.0 µm and a GSD less than 1.8, or optimally an MMAD less than 3.6 and a GSD less than 1.6. In some embodiments, the therapeutic effect comprises an improvement of FEV₁ above baseline of at least about 10% at 24 hours after the composition is administered with the high efficiency nebulizer. In some embodiments, the therapeutic effect comprises an improvement of FEV₁ above baseline of at least about 100 mL at 24 hours after the composition is administered with the high efficiency nebulizer. In some embodiments, the composition further comprises a beta 2-adrenoreceptor agonist, a corticosteroid, or both.

Some embodiments described herein provide a method of treating a patient having chronic obstructive pulmonary disease (COPD), comprising administering to the patient, with a high efficiency nebulizer, a nominal dose calculated to produce a respirable dose of a composition comprising a muscarinic antagonist, wherein producing said calculated respirable dose with said high efficiency nebulizer provides to the patient: (1) at least similar magnitude and/or duration of therapeutic effect; and (2) reduced side effects, compared to administering a nominal dose calculated to produce substantially the same respirable dose of the muscarinic antagonist with a conventional nebulizer. In some embodiments, the composition comprising the muscarinic antagonist is a concentrated, preservative-free, pH-adjusted solution formulation of the muscarinic antagonist. In some embodiments, the concentration of the muscarinic antagonist is greater than about 0.25 mg/mL, greater than about 0.5 mg/mL or greater than about 1 mg/mL. In some embodiments, the composition has a pH of 3 to 5. In some embodiments, the formulation is room temperature stable for at least 2 years. In some embodiments, the composition comprising the muscarinic antagonist contains about 50 µg to about 1000 µg of glycopyrrolate as the muscarinic antagonist. In some embodiments, the composition has a volume of about 1.0 mL or less, or optimally 0.5 mL or less. In some embodiments, the composition is administered in about 3 minutes or less. In some embodiments, the solution has a stabilizing excipient. In some embodiments, the stabilizing excipient is ethylenediaminetetraacetic acid (EDTA) or a pharmaceutically acceptable salt thereof. In some embodiments, the composition further comprises an excipient to mitigate side effects, for example dry mouth. In some embodiments, the excipient comprises citric acid or a pharmaceutically acceptable salt thereof. In some embodiments, the muscarinic antagonist is a long-acting muscarinic antagonist. In some embodiments, the nominal dose of the composition comprising the muscarinic antagonist contains about 50 µg to about 1000 µg of glycopyrrolate as the muscarinic antagonist. In some embodiments, the nominal dose of the composition comprising the muscarinic antagonist contains about 50 µg to about 500 µg of glycopyrrolate as the muscarinic antagonist. In some embodiments, the composition comprising the muscarinic antagonist contains about 50 µg to about 300 µg of glycopyrrolate as the muscarinic antagonist. In some embodiments, the composition comprising the muscarinic antagonist contains about 50 µg to about 150 µg of glycopyrrolate as the muscarinic antagonist. In some embodiments, the high efficiency nebulizer emits droplets having a Mass Median Aerodynamic Diameter (MMAD) of less than about 4.5 µm and a geometric standard deviation (GSD) of less than about 2.0, an MMAD of less than 4.0 µm and a GSD less than 1.8, or optimally an MMAD less than 3.6 and a GSD less than 1.6. In some embodiments, the therapeutic effect comprises an improvement of FEV₁ above baseline of at least about 10% at 24 hours after the composition is administered with the high efficiency nebulizer. In some embodiments, the therapeutic effect comprises an improvement of FEV₁ above baseline of at least about 100 mL at 24 hours after the composition is administered with the high efficiency nebulizer. In some embodiments, the composition further comprises a beta 2-adrenoreceptor agonist, a corticosteroid, or both.

Some embodiments disclosed herein provide a method of treating a patient having chronic obstructive pulmonary disease (COPD), comprising administering to the patient, with a high efficiency nebulizer, a nominal dose that achieves a deposited lung dose of a composition comprising glycopyrrolate, wherein administering said nominal dose with said high efficiency nebulizer provides to the patient: (1) at least similar magnitude and/or duration of therapeutic effect; and (2) reduced side effects, compared to administering a nominal dose that achieves the same deposited lung dose of composition comprising glycopyrrolate with a conventional nebulizer. Some embodiments provide a unit dosage form, which comprises a container that holds a nominal dose of a composition comprising a muscarinic antagonist, such as glycopyrrolate, said container being adapted or adaptable to operate with a high efficiency nebulizer to carry out the foregoing method. Some embodiments provide a combination of (1) a high efficiency nebulizer and (2) the aforementioned unit dosage form. In some embodiments, an effective dose is about 0.05 to about 1.0 mg of glycopyrrolate. In some embodiments, an effective dose is about 0.05 to about 0.5 mg of glycopyrrolate. In some embodiments, an effective dose is about 0.05 to about 0.125 mg of glycopyrrolate. In some embodiments, an effective dose is about 0.05 to about 0.1 mg of glycopyrrolate. In some embodiments, an effective dose is less than 0.1 mg, especially less than or equal to about 0.08 mg of glycopyrrolate. In some embodiments, the composition has a glycopyrrolate concentration of about 0.1 mg/ml to about 2 mg/ml. In some embodiments, the nominal dose has a fill volume of about 0.5 ml. In some embodiments, said nominal dose is administrable with said high efficiency nebulizer in about 3 min or less. In some embodiments, said composition is preservative free. In some embodiments, said composition contains a stabilizing excipient. In some embodiments, said stabilizing excipient is EDTA. In some embodiments, said composition contains an excipient or drug to mitigate at least one side effect. In some embodiments, said excipient or drug reduces dry mouth. In some embodiments, said excipient or drug is a sialogogic agent. In some embodiments, the sialogogic agent is citric acid, other pharmaceutically acceptable acid or salt or mixture thereof.

A method of treating a patient having chronic obstructive pulmonary disease (COPD), comprising administering to the patient, with a high efficiency nebulizer, a nominal dose of a composition containing less than about 100 µg of glycopyrrolate, whereby said patient experiences a therapeutic effect, with therapeutically acceptable side effects, for at least about 12 hours, at least about 18 hours, at least about 24 hours, at least about 30 hours or at least about 36 hours. In some embodiments, said patient experiences a therapeutic effect for at least about 18 hours. In some embodiments, said patient experiences a therapeutic effect for at least about 24 hours. In some embodiments, the patient experiences a therapeutic effect for at least about 30 hours. In some embodiments, the patient experiences a therapeutic effect for at least about 36 hours. In some embodiments, the composition comprising the muscarinic antagonist is a concentrated, preservative-free, pH-adjusted solution formulation of the muscarinic antagonist. In some embodiments, the nominal dose of the composition comprising the muscarinic antagonist contains about 50 µg to about 100 µg of glycopyrrolate as the muscarinic antagonist. In some embodiments, the high efficiency nebulizer emits droplets having a Mass Median Aerodynamic Diameter (MMAD) of less than about 4.5 µm and a geometric standard deviation (GSD) of less than about 2.0. In some embodiments, the therapeutic effect comprises an improvement of FEV₁ above baseline of at least about 10% at 24 hours after administration of the composition with the high efficiency nebulizer. In some embodiments, the therapeutic effect comprises an improvement of FEV₁ above baseline of at least about 100 mL at 24 hours after the composition is administered with the high efficiency nebulizer. In some embodiments, the composition further comprises a beta 2-adrenoreceptor agonist, a corticosteroid, or both.

Some embodiments described herein provide a method of treating a patient having chronic obstructive pulmonary disease (COPD), comprising administering to the patient, with a high efficiency nebulizer, a nominal dose of a composition comprising glycopyrrolate, wherein administering said nominal dose with said high efficiency nebulizer provides to the patient a calculated respirable dose of said composition, wherein said respirable dose with said high efficiency nebulizer provides the patient with a reduction of severity, duration, and/or frequency of one or more side effects, compared to administering a nominal dose calculated to produce substantially the same respirable dose of glycopyrrolate with a conventional nebulizer. In some embodiments, the composition comprising the muscarinic antagonist is a concentrated, preservative-free, pH-adjusted solution formulation of the muscarinic antagonist. In some embodiments, the concentration of the muscarinic antagonist is greater than about 0.25 mg/mL, greater than about 0.5 mg/mL or greater than about 1 mg/mL. In some embodiments, the composition has a pH of 3 to 5. In some embodiments, the formulation is room temperature stable for at least 2 years. In some embodiments, the composition comprising the muscarinic antagonist contains about 50 µg to about 1000 µg of glycopyrrolate as the muscarinic antagonist. In some embodiments, the composition has a volume of about 1.0 mL or less, or optimally 0.5 mL or less. In some embodiments, the composition is administered in about 3 minutes or less. In some embodiments, the solution has a stabilizing excipient. In some embodiments, the stabilizing excipient is ethylenediaminetetraacetic acid (EDTA) or a pharmaceutically acceptable salt thereof. In some embodiments, the composition further comprises an excipient to mitigate side effects, for example dry mouth. In some embodiments, the excipient comprises citric acid or a pharmaceutically acceptable salt thereof. In some embodiments, the muscarinic antagonist is a long-acting muscarinic antagonist. In some embodiments, the nominal dose of the composition comprising the muscarinic antagonist contains about 50 µg to about 1000 µg of glycopyrrolate as the muscarinic antagonist. In some embodiments, the nominal dose of the composition comprising the muscarinic antagonist contains about 50 µg to about 500 µg of glycopyrrolate as the muscarinic antagonist. In some embodiments, the composition comprising the muscarinic antagonist contains about 50 µg to about 300 µg of glycopyrrolate as the muscarinic antagonist. In some embodiments, the composition comprising the muscarinic antagonist contains about 50 µg to about 150 µg of glycopyrrolate as the muscarinic antagonist. In some embodiments, the high efficiency nebulizer emits droplets having a Mass Median Aerodynamic Diameter (MMAD) of less than about 4.5 µm and a geometric standard deviation (GSD) of less than about 2.0, an MMAD of less than 4.0 µm and a GSD less than 1.8, or optimally an MMAD less than 3.6 and a GSD less than 1.6. In some embodiments, the therapeutic effect comprises an improvement of FEV₁ above baseline of at least about 10% at 24 hours after the composition is administered with the high efficiency nebulizer. In some embodiments, the therapeutic effect comprises an improvement of FEV₁ above baseline of at least about 100 mL at 24 hours after the composition is administered with the high efficiency nebulizer. In some embodiments, the composition further comprises a beta 2-adrenoreceptor agonist, a corticosteroid, or both.

Some embodiments described herein provide a method of treating a patient having chronic obstructive pulmonary disease (COPD), comprising administering to the patient, with a high efficiency nebulizer, a nominal dose of a composition comprising a muscarinic antagonist, wherein administering said nominal dose with said high efficiency nebulizer achieves in the patient a deposited lung dose of said composition, wherein achievement of said deposited lung dose with said high efficiency nebulizer provides the patient with a reduction of severity, duration, and/or frequency of one or more side effects, compared to administering a nominal dose that achieves substantially the same deposited lung dose of the muscarinic antagonist with a conventional nebulizer. In some embodiments, the composition comprising the muscarinic antagonist is a concentrated, preservative-free, pH-adjusted solution formulation of the muscarinic antagonist. In some embodiments, the concentration of the muscarinic antagonist is greater than about 0.25 mg/mL, greater than about 0.5 mg/mL or greater than about 1 mg/mL. In some embodiments, the composition has a pH of 3 to 5. In some embodiments, the composition comprising the muscarinic antagonist contains about 50 µg to about 1000 µg of glycopyrrolate as the muscarinic antagonist. In some embodiments, the composition has a volume of about 1.0 mL or less, or optimally 0.5 mL or less. In some embodiments, the composition is administered in about 3 minutes or less. In some embodiments, the solution has a stabilizing excipient. In some embodiments, the stabilizing excipient is ethylenediaminetetraacetic acid (EDTA) or a pharmaceutically acceptable salt thereof. In some embodiments, the composition further comprises an excipient to mitigate side effects, for example dry mouth. In some embodiments, the excipient comprises citric acid or a pharmaceutically acceptable salt thereof. In some embodiments, the muscarinic antagonist is a long-acting muscarinic antagonist. In some embodiments, the nominal dose of the composition comprising the muscarinic antagonist contains about 50 µg to about 1000 µg of glycopyrrolate as the muscarinic antagonist. In some embodiments, the nominal dose of the composition comprising the muscarinic antagonist contains about 50 µg to about 500 µg of glycopyrrolate as the muscarinic antagonist. In some embodiments, the composition comprising the muscarinic antagonist contains about 50 µg to about 300 µg of glycopyrrolate as the muscarinic antagonist. In some embodiments, the composition comprising the muscarinic antagonist contains about 50 µg to about 150 µg of glycopyrrolate as the muscarinic antagonist. In some embodiments, the high efficiency nebulizer emits droplets having a Mass Median Aerodynamic Diameter (MMAD) of less than about 4.5 µm and a geometric standard deviation (GSD) of less than about 2.0, an MMAD of less than 4.0 µm and a GSD less than 1.8, or optimally an MMAD less than 3.6 and a GSD less than 1.6. In some embodiments, the therapeutic effect comprises an improvement of FEV₁ above baseline of at least about 10% at 24 hours after the composition is administered with the high efficiency nebulizer. In some embodiments, the therapeutic effect comprises an improvement of FEV₁ above baseline of at least about 100 mL at 24 hours after the composition is administered with the high efficiency nebulizer. In some embodiments, the composition further comprises a beta 2-adrenoreceptor agonist, a corticosteroid, or both.

Some embodiments described herein provide a method of treating a patient having chronic obstructive pulmonary disease (COPD), comprising administering to the patient, with a nebulizer, a nominal dose of a composition comprising a muscarinic antagonist that is effective to provide the patient with a therapeutic effect for at least about 24 hours. In some embodiments, the method comprises administering said nominal dose with the nebulizer produces in the patient therapeutically acceptable side effects. In some embodiments, the composition comprising the muscarinic antagonist is a concentrated, preservative-free, pH-adjusted solution formulation of the muscarinic antagonist. In some embodiments, the composition comprising the muscarinic antagonist contains about 50 µg to about 1000 µg of glycopyrrolate as the muscarinic antagonist. In some embodiments, the muscarinic antagonist is a long-acting muscarinic antagonist. In some embodiments, the nominal dose of the composition comprising the muscarinic antagonist contains about 50 µg to about 2000 µg of glycopyrrolate as the muscarinic antagonist. In some embodiments, the nominal dose of the composition comprising the muscarinic antagonist contains about 50 µg to about 500 µg of glycopyrrolate as the muscarinic antagonist. In some embodiments, the composition comprising the muscarinic antagonist contains about 50 µg to about 300 µg of glycopyrrolate as the muscarinic antagonist. In some embodiments, the composition comprising the muscarinic antagonist contains about 50 µg to about 150 µg of glycopyrrolate as the muscarinic antagonist. In some embodiments, the high efficiency nebulizer emits droplets having a Mass Median Aerodynamic Diameter (MMAD) of less than about 4.5 µm and a geometric standard deviation (GSD) of less than about 2.0, an MMAD of less than 4.0 µm and a GSD less than 1.8, or optimally an MMAD less than 3.6 and a GSD less than 1.6. In some embodiments, the therapeutic effect comprises an improvement of FEV₁ above baseline of at least about 10% at 24 hours after administration of the composition with the high efficiency nebulizer. In some embodiments, the therapeutic effect comprises an improvement of FEV₁ above baseline of at least about 100 mL at 24 hours after the composition is administered with the high efficiency nebulizer. In some embodiments, the composition further comprises a beta 2-adrenoreceptor agonist, a corticosteroid, or both.

Some embodiments described herein provide a composition for administration with a high efficiency nebulizer, comprising a concentrated, preservative-free, pH-adjusted solution formulation of the muscarinic antagonist. In some embodiments, the muscarinic antagonist is glycopyrrolate. In some embodiments, the glycopyrrolate has a concentration of at least 0.25 mg/mL. In some embodiments, the glycopyrrolate has a concentration of at least 0.5 mg/mL. In some embodiments, the glycopyrrolate has a concentration of at least 1 mg/mL. In some embodiments, the pH is about 3 to about 5. In some embodiments, composition further comprises a beta 2-adrenoreceptor agonist, a corticosteroid, or both.

Some embodiments described herein provide for use of a muscarinic antagonist in the manufacture of a medicament for the treatment of chronic obstructive pulmonary disease (COPD) wherein the medicament is administered from a high efficiency nebulizer. In some embodiments, the muscarinic antagonist is glycopyrrolate. In some embodiments, the glycopyrrolate has a concentration of at least 0.25 mg/mL. In some embodiments, the glycopyrrolate has a concentration of at least 0.5 mg/mL. In some embodiments, the glycopyrrolate has a concentration of at least 1 mg/mL. In some embodiments, the pH is about 3 to about 5. In some embodiments, the medicament is administered from a nebulizer in droplets having a mass median aerodynamic diameter (MMAD) of less than about 4.5 µm and geometric standard deviation (GSD) of about 2 or less. In some embodiments, the medicament further comprises a beta 2-adrenoreceptor agonist, a corticosteroid, or both.

Some embodiments described herein provide a medicament comprising a muscarinic antagonist for the treatment of COPD by administration from a high efficiency nebulizer. In some embodiments, the muscarinic antagonist is glycopyrrolate. In some embodiments, the glycopyrrolate has a concentration of at least 0.25 mg/mL. In some embodiments, the glycopyrrolate has a concentration of at least 0.5 mg/mL. In some embodiments, the glycopyrrolate has a concentration of at least 1 mg/mL. In some embodiments, the pH is about 3 to about 5. In some embodiments, the medicament is administered from a nebulizer in droplets having a mass median aerodynamic diameter (MMAD) of less than about 4.5 µm and geometric standard deviation (GSD) of about 2 or less. In some embodiments, the medicament further comprises a beta 2-adrenoreceptor agonist, a corticosteroid, or both.

Some embodiments disclosed herein provide a method of treating a patient having chronic obstructive pulmonary disease (COPD), comprising administering to the patient, with a nebulizer, a nominal dose of a composition comprising glycopyrrolate that is effective to provide the patient with a therapeutic effect for at least about 24 hours. Some embodiments provide a unit dosage form, which comprises a container that holds a nominal dose of a composition comprising a muscarinic antagonist, such as glycopyrrolate, said container being adapted or adaptable to operate with a high efficiency nebulizer to carry out the foregoing method. Some embodiments provide a device comprising a combination of (1) a high efficiency nebulizer and (2) the aforementioned unit dosage form. In some embodiments, administering said nominal dose of said composition with the nebulizer produces in the patient no more than therapeutically acceptable side effects. In some embodiments, the composition contains glycopyrrolate as the muscarinic antagonist and has a glycopyrrolate concentration of about 0.1 mg/ml to about 2 mg/ml. In some embodiments, the nominal dose has a fill volume of about 0.5 ml. In some embodiments, said nominal dose is administrable with said nebulizer in about 10 min. or less, about 7 min. or less, about 5 min. or less, or about 3 min. or less. In some embodiments, said composition is preservative free. In some embodiments, said composition contains a stabilizing excipient. In some embodiments, said stabilizing excipient is EDTA. In some embodiments, said composition contains an excipient or drug to mitigate at least one side effect. In some embodiments, said excipient or drug reduces dry mouth. In some embodiments, said excipient or drug is a sialogogic agent. In some embodiments, sialogogic agent is citric acid, other pharmaceutically acceptable acid or salt or mixture thereof. In some embodiments, the nominal dose is administered with a high efficiency nebulizer. In some embodiments, the nominal dose is administered with a conventional nebulizer. In some embodiments, an effective dose is about 0.05 to about 0.5 mg of glycopyrrolate. In some embodiments, an effective dose is about 0.05 to about 0.125 mg of glycopyrrolate. In some embodiments, an effective dose is about 0.05 to about 0.1 mg of glycopyrrolate. In some embodiments, an effective dose is less than 0.1 mg, especially less than or equal to about 0.08 mg of glycopyrrolate.

Some embodiments disclosed herein provide a method of treating a patient having chronic obstructive pulmonary disease (COPD), comprising administering to the patient, with a high efficiency nebulizer, a nominal dose of a composition comprising a muscarinic antagonist, whereby the patient experiences a therapeutic effect for at least about 24 hours. Some embodiments provide a unit dosage form, which comprises a container that holds a nominal dose of a composition comprising a muscarinic antagonist, such as glycopyrrolate, said container being adapted or adaptable to operate with a high efficiency nebulizer to carry out the foregoing method. Some embodiments provide a device that comprises a combination of (1) a high efficiency nebulizer and (2) the aforementioned unit dosage form. In some embodiments, the patient experiences no more than therapeutically acceptable side effects. In some embodiments, administering said nominal dose of the composition with the high efficiency nebulizer provides the patient with reduced side effects compared to administering the same nominal dose with a conventional nebulizer. In some embodiments, administering said nominal dose of the composition with the high efficiency nebulizer achieves in the patient a calculated respirable dose of glycopyrrolate, whereby the patient experiences reduced side effects compared to achieving the same calculated respirable dose with a conventional nebulizer. In some embodiments, administering said nominal dose of the composition with the high efficiency nebulizer achieves in the patient a deposited lung dose of glycopyrrolate, whereby the patient experiences reduced side effects compared to achieving the same deposited lung dose with a conventional nebulizer. In some embodiments, the composition administered with the high efficiency nebulizer has a muscarinic antagonist concentration of at least 2 times, 3 times or 4 times the concentration of the same nominal dose of the muscarinic antagonist with a conventional nebulizer. In some embodiments, the muscarinic antagonist is glycopyrrolate and has a concentration of 0.1 mg/ml to about 2 mg/ml. In some embodiments, the nominal dose has a fill volume of about 0.5 ml. In some embodiments, said nominal dose is administrable with said high efficiency nebulizer in about 3 min or less. In some embodiments, said composition is preservative free. In some embodiments, said composition contains a stabilizing excipient. In some embodiments, said stabilizing excipient is EDTA. In some embodiments, said composition contains an excipient or drug to mitigate at least one side effect. In some embodiments, excipient reduces dry mouth. In some embodiments, said excipient or drug is a sialogogic agent. In some embodiments, the sialogogic agent is citric acid, other pharmaceutically acceptable acid or salt or mixture thereof. In some embodiments, the muscarinic antagonist is selected from the group of long-acting muscarinic antagonists (LAMAs) and short-acting muscarinic antagonists (SAMAs). In some embodiments, the muscarinic antagonist is a long-acting muscarinic antagonist (LAMA). In some embodiments, the LAMA is selected from the group consisting of: glycopyrrolate, tiotropium, aclidinium, trospium, QAT 370, GSK, 233705, GSK 656398, BEA 218 or a pharmaceutical acceptable derivative, salt, enantiomer, diastereomer, or racemic mixture thereof. In some embodiments, the muscarinic antagonist is a short-acting muscarinic antagonist (SAMA). In some embodiments, the SAMA is selected from the group consisting of: ipratropium, oxitropium, or a pharmaceutical acceptable derivative, salt, enantiomer, diastereomer, or racemic mixture thereof.

Some embodiments disclosed herein provide a method of treating a patient having chronic obstructive pulmonary disease (COPD), comprising administering to the patient, with a high efficiency nebulizer, a nominal dose of a composition comprising a muscarinic antagonist, whereby the patient experiences: (1) an increased magnitude and/or duration of therapeutic effect; and (2) reduced side effects, compared to administering the same nominal dose of composition comprising a muscarinic antagonist with a conventional nebulizer. In some embodiments, the composition administered with the high efficiency nebulizer has a muscarinic antagonist concentration of at least 2 times, 3 times or 4 times the concentration of the same nominal dose of the muscarinic antagonist with a conventional nebulizer. In some embodiments, the muscarinic antagonist is glycopyrrolate and has a concentration of 0.1 mg/ml to about 2 mg/ml. Some embodiments provide a unit dosage form, which comprises a container that holds a nominal dose of a composition comprising a muscarinic antagonist, such as glycopyrrolate, said container being adapted or adaptable to operate with a high efficiency nebulizer to carry out the foregoing method. Some embodiments provide a combination of (1) a high efficiency nebulizer and (2) the aforementioned unit dosage form. In some embodiments, the nominal dose has a fill volume of about 0.5 ml. In some embodiments, said nominal dose is administrable with said high efficiency nebulizer in about 3 min or less. In some embodiments, said composition is preservative free. In some embodiments, said composition contains a stabilizing excipient. In some embodiments, said stabilizing excipient is EDTA. In some embodiments, said composition contains an excipient or drug to mitigate at least one side effect. In some embodiments, said excipient or drug reduces dry mouth. In some embodiments, said excipient or drug is a sialogogic agent. In some embodiments, the sialogogic agent is citric acid, other pharmaceutically acceptable acid or salt or mixture thereof. In some embodiments, the muscarinic antagonist is selected from the group of long-acting muscarinic antagonists (LAMAs) and short-acting muscarinic antagonists (SAMAs). In some embodiments, the muscarinic antagonist is a long-acting muscarinic antagonist (LAMA). In some embodiments, LAMA is selected from the group consisting of: glycopyrrolate, tiotropium, aclidinium, trospium, QAT 370, GSK, 233705, GSK 656398, BEA 218 or a pharmaceutical acceptable derivative, salt, enantiomer, diastereomer, or racemic mixture thereof. In some embodiments, the muscarinic antagonist is a short-acting muscarinic antagonist (SAMA). In some embodiments, the SAMA is selected from the group consisting of: ipratropium, oxitropium, or a pharmaceutical acceptable derivative, salt, enantiomer, diastereomer, or racemic mixture thereof.

Some embodiments disclosed herein provide a method of treating a patient having chronic obstructive pulmonary disease (COPD), comprising administering to the patient, with a high efficiency nebulizer, a nominal dose to achieve a calculated respirable dose of a composition comprising a muscarinic antagonist, whereby the patient experiences: (1) an increased magnitude and/or duration of therapeutic effect; and (2) reduced side effects, compared to administering the same respirable dose of composition comprising a muscarinic antagonist with a conventional nebulizer. Some embodiments provide a unit dosage form, which comprises a container that holds a nominal dose of a composition comprising a muscarinic antagonist, such as glycopyrrolate, said container being adapted or adaptable to operate with a high efficiency nebulizer to carry out the foregoing method. Some embodiments provide a combination of (1) a high efficiency nebulizer and (2) the aforementioned unit dosage form. In some embodiments, the composition administered with the high efficiency nebulizer has a muscarinic antagonist concentration of at least 2 times, 3 times or 4 times the concentration of the same nominal dose of the muscarinic antagonist with a conventional nebulizer. In some embodiments, the muscarinic antagonist is glycopyrrolate and has a concentration of 0.1 mg/ml to about 2 mg/ml. In some embodiments, the nominal dose has a fill volume of about 0.5 ml. In some embodiments, said nominal dose is administrable with said high efficiency nebulizer in about 3 min or less. In some embodiments, said composition is preservative free. In some embodiments, said composition contains a stabilizing excipient. In some embodiments, said stabilizing excipient is EDTA. In some embodiments, said composition contains an excipient or drug to mitigate at least one side effect. In some embodiments, said excipient or drug reduces dry mouth. In some embodiments, said excipient or drug is a sialogogic agent. In some embodiments, the sialogogic agent is citric acid, other pharmaceutically acceptable acid or salt or mixture thereof. In some embodiments, the muscarinic antagonist is selected from the group of long-acting muscarinic antagonists (LAMAs) and short-acting muscarinic antagonists (SAMAs). In some embodiments, the muscarinic antagonist is a long-acting muscarinic antagonist (LAMA). In some embodiments, the LAMA is selected from the group consisting of: glycopyrrolate, tiotropium, aclidinium, trospium, QAT 370, GSK, 233705, GSK 656398, BEA 218 or a pharmaceutical acceptable derivative, salt, enantiomer, diastereomer, or racemic mixture thereof. In some embodiments, the muscarinic antagonist is a short-acting muscarinic antagonist (SAMA). In some embodiments, the SAMA is selected from the group consisting of: ipratropium, oxitropium, or a pharmaceutical acceptable derivative, salt, enantiomer, diastereomer, or racemic mixture thereof.

Some embodiments described herein provide a method for the treatment of COPD in a patient, comprising administering to the patient a nominal dose of a muscarinic antagonist in an aqueous inhalation solution with a high efficiency nebulizer, wherein administration of the muscarinic antagonist with the inhalation device provides muscarinic antagonist lung deposition (deposited lung dose) of at least about at least about 30%, at least about 35%, at least about 40%, at least about 45%, at least about 50%, at least about 55%, at least about 60%, about 30% to about 60%, about 30% to about 55%, about 30% to about 50%, about 30% to about 40%, about 30% to about 75%, about 40% to about 70%, or about 45% to about 60%, of the nominal dose of the muscarinic antagonist. Some embodiments provide a unit dosage form, which comprises a container that holds a nominal dose of a composition comprising a muscarinic antagonist, such as glycopyrrolate, said container being adapted or adaptable to operate with a high efficiency nebulizer to carry out the foregoing method. Some embodiments provide a combination of (1) a high efficiency nebulizer and (2) the aforementioned unit dosage form.

Some embodiments described herein provide an inhalation system for the treatment or prophylaxis of a respiratory condition in a patient, the system comprising: (a) a nominal dose of a muscarinic antagonist in an aqueous inhalation solution; and (b) a high efficiency nebulizer, wherein administration of the muscarinic antagonist with the inhalation device provides muscarinic antagonist lung deposition (deposited lung dose) of at least about 30%, at least about 35%, at least about 40%, at least about 45%, at least about 50%, at least about 55%, at least about 60%, about 30% to about 60%, about 30% to about 55%, about 30% to about 50%, about 30% to about 40%, about 30% to about 75%, about 40% to about 70%, or about 45% to about 60%, of the nominal dose of the muscarinic antagonist. Some embodiments provide a unit dosage form, which comprises a container that holds a nominal dose of a composition comprising a muscarinic antagonist, such as glycopyrrolate, said container being adapted or adaptable to operate with a high efficiency nebulizer to carry out the foregoing method. Some embodiments provide a combination of (1) a high efficiency nebulizer and (2) the aforementioned unit dosage form.

Some embodiments described herein provide a method for the treatment of COPD in a patient, comprising administering to the patient a nominal dose of a muscarinic antagonist (e.g. glycopyrrolate) in an aqueous inhalation solution with a high efficiency nebulizer, wherein administration of the muscarinic antagonist with the inhalation device provides one or both of (a) and (b); and wherein administration of the muscarinic antagonist with the inhalation device provides one or more of (i), (ii) and (iii): (a) a respirable dose delivery rate (RDDR) of at least about 2 times, 3 times or 4 times the RDDR achievable with a conventional nebulizer (for example, where the muscarinic antagonist is glycopyrrolate, in some embodiments, RDDR is at least about 100 µg/min, at least about 150 µg/min, at least about 200 µg/min, about 100 µg/min to about 5,000 µg/min, about 150 µg/min to about 4,000 µg/min or about 200 µg/min to about 3,500 µg/min); (b) an output rate of muscarinic antagonist of at least about 2 times, 3 times or 4 times the output rate achievable with a conventional nebulizer (for example, where the muscarinic antagonist is glycopyrrolate, the output rate is at least about 120 µg/min, at least about 150 µg/min, at least about 200 µg/min or at least about 200 µg/min to about 5,000 µg/min); (i) a respirable fraction (RF) of muscarinic antagonist of at least about 60%, at least about 65%, at least about 70%, at least about 75%, at least about 80%, at least about 85%, at least about 90%, about 60% to about 95%, about 65% to about 95%, about 65% to about 90% or about 70% to about 90%; (ii) a Geometric Standard Deviation (GSD) of emitted droplet size distribution of the solution administered with a high efficiency nebulizer of about 1.1 to about 2.1, about 1.2 to about 2.0, about 1.3 to about 1.9, about 1.4 to about 1.8, about 1.5 to about 1.7, about 1.4, about 1.5, or about 1.6; (iii) a Mass Median Aerodynamic Diameter (MMAD) of droplet size of the solution emitted with the high efficiency nebulizer of about 1 µm to about 5 µm, about 2 µm to about 4 µm, about 3 µm to about 4 µm, or about 3.5 µm to about 4.5 µm.

Some embodiments described herein provide an inhalation system for the treatment or prophylaxis of a respiratory condition in a patient, the system comprising: a nominal dose of a muscarinic antagonist in an aqueous inhalation solution; and a high efficiency nebulizer, wherein administration of the muscarinic antagonist with the inhalation device provides one or both of (a) and (b); and wherein administration of the muscarinic antagonist with the inhalation device provides one or more of (i), (ii) and (iii): (a) a respirable dose delivery rate (RDDR) of at least about 2 times, 3 times or 4 times the RDDR achievable with a conventional nebulizer (for example, where the muscarinic antagonist is glycopyrrolate, in some embodiments, RDDR is at least about 100 µg/min, at least about 150 µg/min, at least about 200 µg/min, about 100 µg/min to about 5,000 µg/min, about 150 µg/min to about 4,000 µg/min or about 200 µg/min to about 3,500 µg/min); (b) an output rate of muscarinic antagonist of at least about 2 times, 3 times or 4 times the output rate achievable with a conventional nebulizer (for example, where the muscarinic antagonist is glycopyrrolate, the output rate is at least about 120 µg/min, at least about 150 µg/min, at least about 200 µg/min or at least about 200 µg/min to about 5,000 µg/min); (i) a respirable fraction (RF) of muscarinic antagonist of at least about 60%, at least about 65%, at least about 70%, at least about 75%, at least about 80%, at least about 85%, at least about 90%, about 60% to about 95%, about 65% to about 95%, about 65% to about 90% or about 70% to about 90%; (ii) a Geometric Standard Deviation (GSD) of emitted droplet size distribution of the solution administered with a high efficiency nebulizer of about 1.1 to about 2.1, about 1.2 to about 2.0, about 1.3 to about 1.9, about 1.4 to about 1.8, about 1.5 to about 1.7, about 1.4, about 1.5, or about 1.6; (iii) a Mass Median Aerodynamic Diameter (MMAD) of droplet size of the solution emitted with the high efficiency nebulizer of about 1 µm to about 5 µm, about 2 to about 4 µm, about 3 µm to about 4 µm, about 3 to about 4 µm, or about 3.5 to about 4.5 µm. Some embodiments provide a unit dosage form, which comprises a container that holds a nominal dose of a composition comprising a muscarinic antagonist, such as glycopyrrolate, said container being adapted or adaptable to operate with a high efficiency nebulizer to carry out the foregoing method. Some embodiments provide a combination of (1) a high efficiency nebulizer and (2) the aforementioned unit dosage form.

Some embodiments described herein provide a method for the treatment of COPD in a patient, the method comprising administering to the patient a nominal dose of a muscarinic antagonist in an aqueous inhalation solution with an inhalation device, wherein the aqueous inhalation solution has a muscarinic antagonist concentration of about 2 times, about 3 times, or about 4 times the concentration of an aqueous inhalation solution of the same muscarinic antagonist in an inhalation solution for use with a conventional nebulizer, (for example, when the muscarinic antagonist is glycopyrrolate, the concentration is in some embodiments about 0.25 mg/mL to about 50 mg/mL, about 0.25 mg/mL to about 20 mg/mL, about 0.25 mg/mL to about 10 mg/mL, about 0.5 mg/mL to about 50 mg/mL, about 0.5 mg/mL to about 20 mg/mL, about 0.5 to about 10 mg/mL, at least about 0.5 mg/mL, at least about 1.0 mg/mL, at least about 1.5 mg/mL, at least about 2.0 mg/mL, at least about 5 mg/mL, at least about 10 mg/mL, at least about 20 mg/mL or at least about 25 mg/mL). In some such embodiments, the muscarinic antagonist is glycopyrrolate.

Some embodiments described herein provide an inhalation system for the treatment or prophylaxis of a respiratory condition in a patient, the system comprising: (a) a nominal dose of a muscarinic antagonist in an aqueous inhalation solution; and (b) an inhalation device, wherein the aqueous inhalation solution the aqueous inhalation solution has a muscarinic antagonist concentration of at least about 2 times, about 3 times or about 4 times a concentration of the same muscarinic antagonist for use in a conventional nebulizer (for example, when the muscarinic antagonist is glycopyrrolate, in some embodiments the concentration of glycopyrrolate is 0.25 mg/mL to about 50 mg/mL, about 0.25 mg/mL to about 20 mg/mL, about 0.25 mg/mL to about 10 mg/mL, about 0.5 mg/mL to about 50 mg/mL, about 0.5 mg/mL to about 20 mg/mL, about 0.5 to about 10 mg/mL, at least about 0.5 mg/mL, at least about 1.0 mg/mL, at least about 1.5 mg/mL, at least about 2.0 mg/mL, at least about 5 mg/mL, at least about 10 mg/mL, at least about 20 mg/mL or at least about 25 mg/mL).

Some embodiments described herein provide a method for the treatment of COPD in a patient, the method comprising administering to the patient a nominal dose of a muscarinic antagonist in an aqueous inhalation solution with an inhalation device, wherein the aqueous inhalation solution provides a duration of therapeutic effect of at least about 12 hr, about 12 hr to about 24 hr, about 18 hr to about 24 hr, about 20 hr to about 24 hr, or at least about 24 hr.

Some embodiments described herein provide an inhalation system for the treatment or prophylaxis of a respiratory condition in a patient, the system comprising: (a) a nominal dose of a muscarinic antagonist in an aqueous inhalation solution; and (b) an inhalation device, wherein the aqueous inhalation solution provides a duration of therapeutic effect of at least about 12 hr, about 12 hr to about 24 hr, about 18 hr to about 24 hr, about 20 hr to about 24 hr or at least about 24 hr.

Some embodiments described herein provide a method for the treatment of acute exacerbations of COPD in a patient, comprising administering to the patient a nominal dose of muscarinic antagonist in an aqueous inhalation solution at a concentration and delivery rate of muscarinic antagonist sufficient to provide a rapid onset of therapeutic effect and a long duration of therapeutic effect. In some embodiments, the method provides a rapid onset of therapeutic effect. In some embodiments, the onset of therapeutic effect is less than 5 minutes, less than 10 minutes, or about 5-10 minutes. In some embodiments, onset of therapeutic effect is achieved when the patient experiences a clinically relevant improvement. In some embodiments, onset of therapeutic effect is achieved when the patient experiences a clinically relevant improvement in FEV₁. In some embodiments, onset of therapeutic effect is achieved when a patient experiences an increase of FEVI over baseline of at least about 100 mL and/or at least about 10% increase in FEV₁ over baseline. In some embodiments, a device comprises a unit dosage form of a muscarinic antagonist, such as glycopyrrolate, adapted or adaptable for administration with a high efficiency nebulizer.

Some embodiments described herein provide an inhalation system for the treatment of acute exacerbations of COPD in a patient, comprising: (a) a nominal dose of a muscarinic antagonist in an aqueous inhalation solution; and (b) an inhalation device, wherein the muscarinic antagonist has a concentration in the aqueous inhalation solution sufficient to provide a rapid onset of therapeutic effect and a long duration of therapeutic effect. In some embodiments, the method provides a rapid onset of therapeutic effect. In some embodiments, the onset of therapeutic effect is less than 5 minutes, less than 10 minutes, or about 5-10 minutes. In some embodiments, onset of therapeutic effect is achieved when the patient experiences a clinically relevant improvement. In some embodiments, onset of therapeutic effect is achieved when the patient experiences a clinically relevant improvement in FEV₁. In some embodiments, onset of therapeutic effect is achieved when a patient experiences an increase of FEV₁ over baseline of at least about 100 mL and/or at least about 10% increase in FEV₁ over baseline. In some embodiments, a device comprises a unit dosage form of a muscarinic antagonist, such as glycopyrrolate, adapted or adaptable for administration with a high efficiency nebulizer.

Some embodiments described herein provide a method for the treatment of COPD in a patient, the method comprising administering to the patient, with an inhalation device, a nominal dose of a muscarinic antagonist in an aqueous inhalation solution, the aqueous inhalation solution further comprising an excipient or active pharmaceutical ingredient for reducing at least one side effect of the muscarinic antagonist, wherein the aqueous inhalation solution provides a duration of therapeutic effect of at least about 12 hr to about 24 hr, about 18 hr to about 24 hr, about 20 hr to about 24 hr, or at least about 24 hours.

Some embodiments described herein provide an inhalation system for the treatment or prophylaxis of a respiratory condition in a patient, the system comprising: (a) a nominal dose of a muscarinic antagonist in an aqueous inhalation solution further comprising at least one excipient or active pharmaceutical ingredient for reducing at least one side effect of the muscarinic antagonist; and (b) an inhalation device, wherein the aqueous inhalation solution provides a duration of therapeutic effect of at least about 12 hr, about 12 hr to about 24 hr, about 18 hr to about 24 hr, about 20 hr to about 24 hr, or at least about 24 hours.

Some embodiments described herein provide a method for the treatment of COPD in a patient, comprising administering to the patient a nominal dose of muscarinic antagonist which, when administered with a high efficiency nebulizer, provides a calculated respirable dose of a muscarinic antagonist with a high efficiency nebulizer, wherein the calculated respirable dose of the muscarinic antagonist administered with the high efficiency nebulizer demonstrates a decreased incidence and/or severity of systemic and/or local toxicity and/or side effects in the patient as compared to substantially the same calculated respirable dose of the muscarinic antagonist administered with a conventional nebulizer.

Some embodiments described herein provide an inhalation system for the treatment or prophylaxis of a respiratory condition in a patient, the inhalation system comprising: (a) a nominal dose of a muscarinic antagonist, which when administered with a high efficiency nebulizer provides a calculated respirable dose; and (b) a high efficiency nebulizer, wherein the calculated respirable dose of the muscarinic antagonist administered with the high efficiency nebulizer demonstrates a decreased incidence and/or severity of systemic and/or local toxicity and/or side effects in the patient as compared to a nominal dose that achieves substantially the same calculated respirable dose of the muscarinic antagonist with a conventional nebulizer.

Some embodiments described herein provide a method for the treatment of COPD in a patient, comprising administering to the patient a nominal dose of muscarinic antagonist which, when administered with a high efficiency nebulizer, provides a deposited lung dose of a muscarinic antagonist with a high efficiency nebulizer, wherein the deposited lung dose of the muscarinic antagonist administered with the high efficiency nebulizer demonstrates a decreased incidence and/or severity of systemic and/or local toxicity and/or side effects in the patient as compared to a nominal dose that achieves substantially the same deposited lung dose of the muscarinic antagonist administered with a conventional nebulizer.

Some embodiments described herein provide an inhalation system for the treatment or prophylaxis of a respiratory condition in a patient, the inhalation system comprising: (a) a nominal dose of a muscarinic antagonist which, when administered with a high efficiency nebulizer, provides a deposited lung dose; and (b) a high efficiency nebulizer, wherein the deposited lung dose of the muscarinic antagonist administered with the high efficiency nebulizer demonstrates a decreased incidence and/or severity of systemic and/or local toxicity and/or side effects in the patient as compared to substantially the same deposited lung dose of the muscarinic antagonist with a conventional nebulizer.

In some embodiments described, a method or inhalation system of one of the foregoing paragraphs, the muscarinic antagonist is administered with a high efficiency nebulizer selected from the group consisting of a pulsating membrane nebulizer, a nebulizer comprising a vibrating mesh or plate with multiple apertures, a nebulizer comprising a vibration generator and an mixing chamber or a nebulizer that provides: one or both of (a) and (b); and one or more of (i), (ii) and (iii): (a) a respirable dose delivery rate (RDDR) of at least about 2 times, 3 times or 4 times the RDDR achievable with a conventional nebulizer (for example, where the muscarinic antagonist is glycopyrrolate, in some embodiments, RDDR is at least about 100 µg/min, at least about 150 µg/min, at least about 200 µg/min, about 100 µg/min to about 5,000 µg/min, about 150 µg/min to about 4,000 µg/min or about 200 µg/min to about 3,500 µg/min); (b) an output rate of muscarinic antagonist of at least about 2 times, 3 times or 4 times the output rate achievable with a conventional nebulizer (for example, where the muscarinic antagonist is glycopyrrolate, the output rate is at least about 120 µg/min, at least about 150 µg/min, at least about 200 µg/min or at least about 200 µg/min to about 5,000 µg/min); (i) a respirable fraction (RF) of muscarinic antagonist of at least about 60%, at least about 65%, at least about 70%, at least about 75%, at least about 80%, at least about 85%, at least about 90%, about 60% to about 95%, about 65% to about 95%, about 65% to about 90% or about 70% to about 90%; (ii) a Geometric Standard Deviation (GSD) of emitted droplet size distribution of the solution administered with a high efficiency nebulizer of about 1.1 to about 2.1, about 1.2 to about 2.0, about 1.3 to about 1.9, about 1.4 to about 1.8, about 1.5 to about 1.7, about 1.4, about 1.5, or about 1.6; (iii) a Mass Median Aerodynamic Diameter (MMAD) of droplet size of the solution emitted with the high efficiency nebulizer of about 1 µm to about 5 µm, about 2 to about 4 µm, about 3 µm, about 3 to about 4 µm, or about 3.5 to about 4.5 µm.

### DETAILED DESCRIPTION OF THE INVENTION

Unless defined otherwise, all technical and scientific terms used herein have the same meaning as commonly understood by one of skill in the art to which the inventions described herein belong. All publications, patents, and patent applications mentioned in this specification are herein incorporated by reference to the same extent as if each individual publication, patent, or patent application was specifically and individually indicated to be incorporated by reference.

### Definition of Terms

As used herein, the term "about" is used synonymously with the term "approximately." Illustratively, the use of the term "about" with regard to a certain therapeutically effective pharmaceutical dose indicates that values slightly outside the cited values, e.g. plus or minus 0.1% to 10%, which are also effective and safe.

As used herein, the terms "comprising," "including," "such as," and "for example" (or "e.g.") are used in their open, non-limiting sense.

As used herein, the phrase "consisting essentially of' is a transitional phrase used in a claim to indicate that the following list of ingredients, parts or process steps must be present in the claimed composition, machine or process, but that the claim is open to unlisted ingredients, parts or process steps that do not materially affect the basic and novel properties of the invention.

"Nominal dose," as used herein, refers to the loaded dose, which is the amount of active pharmaceutical ingredient ("API") in an inhalation device prior to administration to the patient. The volume of solution containing the nominal dose is referred to as the "fill volume."

"AUCₗₐₛₜ^{HEN}" as used herein, refers to the area under a blood plasma concentration curve up to the last time point for the nominal dose of active pharmaceutical ingredient (API) administered with a high efficiency nebulizer.

"AUCₗₐₛₜ^{Conv}" as used herein, refers to the area under a blood plasma concentration curve up to the last time point for a nominal dose of active pharmaceutical ingredient (API) administered with a conventional nebulizer.

"AUC_{(0-∞)}^{HEN}" as used herein, refers to the area under a blood plasma concentration curve for a nominal dose of active pharmaceutical ingredient (API) administered with a high efficiency nebulizer.

"AUC_{(0-∞)}^{Conv}" as used herein, refers to the area under a blood plasma concentration curve for a nominal dose of active pharmaceutical ingredient (API) administered with a conventional nebulizer [AUC_{(0-∞)}^{Conv}].

"Substantially the same nominal dose" as used herein, means that a first nominal dose of an active pharmaceutical ingredient (API) contains approximately the same number of millimoles of the muscarinic antagonist as a second nominal dose of the muscarinic antagonist.

"Bioavailability" as used herein, refers to the amount of unchanged drug that reaches the systemic circulation. By definition, the bioavailability of an intravenous solution containing the active pharmaceutical ingredient (API) is 100%.

"Enhanced lung deposition," as used herein, refers to an increase in drug deposition (deposited lung dose) arising out of, for example, the improved efficiency of drug delivery with a high efficiency nebulizer. In general, a high efficiency nebulizer will produce a drug cloud having a greater respirable fraction than a conventional nebulizer. While not wishing to be bound by theory, it is considered that a greater respirable fraction will permit greater lung deposition and concomitantly lower oropharyngeal deposition of the drug. In some embodiments, it is considered that reduced oropharyngeal deposition of drug will reduce local side effects, for example dry mouth.

"Deposited dose" or "deposited lung dose" is the amount of muscarinic antagonist deposited in the lung. The deposited dose or deposited lung dose may be expressed in absolute terms, for example the number of µg of API deposited in the lungs. The deposited lung dose may also be expressed in relative terms, for example comparing the mass of API deposited in the lungs with a high efficiency nebulizer to the mass of API deposited in the lungs with a conventional nebulizer.

⁶Cₘₐₓ.^{HEN"} as used herein, refers to the maximum blood plasma concentration for a nominal dose of the active pharmaceutical ingredient (API) administered with a high efficiency nebulizer.

"Cₘₐₓ^{Conv}" as used herein, refers to the maximum blood plasma concentration for a nominal dose of the active pharmaceutical ingredient (API) administered with a conventional nebulizer.

"Enhanced pharmacokinetic profile" means an improvement in some pharmacokinetic parameter. Pharmacokinetic parameters that may be improved include, AUCₗₐₛₜ, AUQ_{(0-∞)} Tₘₐₓ, and optionally a Cₘₐₓ. In some embodiments, the enhanced pharmacokinetic profile may be measured quantitatively by comparing a pharmacokinetic parameter obtained for a nominal dose of an active pharmaceutical ingredient (API) administered with one type of inhalation device (e.g. a high efficiency nebulizer) with the same pharmacokinetic parameter obtained with the same nominal dose of active pharmaceutical ingredient (API) administered with a different type of inhalation device.

"Blood plasma concentration" refers to the concentration of an active pharmaceutical ingredient (API) in the plasma component of blood of a subject or patient population.

"Respiratory condition," as used herein, refers to a disease or condition that is physically manifested in the respiratory tract, including, but not limited to, chronic obstructive pulmonary disease (COPD), bronchitis, chronic bronchitis, emphysema, asthma, or reactive airway disorder (RAD).

"Patient" refers to the animal (especially mammal) or human being treated.

"Muscarinic antagonist" refers to antimuscarinic agents, which are compounds that have the ability to inhibit the action of the neurotransmitter acetylcholine by blocking its binding to muscarinic cholinergic receptors. These agents can be long-acting or short-acting. Long-acting muscarinic antagonists have a therapeutic effect lasting greater than about 6 hours. Some long-acting muscarinic antagonists include, but are not limited to, glycopyrrolate, tiotropium, aclidinium, trospium, QAT 370, GSK, 233705, GSK 656398, BEA 218 or a pharmaceutical acceptable derivative, salt, enantiomer, diastereomer, or racemic mixture thereof. Short-acting muscarinic antagonists have a therapeutic effect for less than about 6 hours. Some short-acting muscarinic antagonists include, but are not limited to, ipratropium, oxitropium, or a pharmaceutical acceptable derivative, salt, enantiomer, diastereomer, or racemic mixture thereof. In some embodiments, the "muscarinic antagonist" is glycopyrrolate, tiotropium, aclidinium, trospium, QAT370, GSK233705, GSK 656398, BEA2180, ipratropium, oxitropium, oxybutynin or a pharmaceutical acceptable derivative, salt, enantiomer, diastereomer, or a pharmaceutical acceptable derivative, salt, enantiomer, diastereomer, or racemic mixture thereof.

"Nebulizer," as used herein, refers to a device that turns medications, compositions, formulations, suspensions, and mixtures, etc. into a fine mist for delivery to the lungs. Nebulizers may also be referred to as atomizers.

"Drug absorption" or simply "absorption" typically refers to the process of movement of drug from site of delivery of a drug across a barrier into a blood vessel or the site of action, e.g., a drug being absorbed in the pulmonary capillary beds of the alveoli.

[Tₘₐₓ^{HEN}] as used herein, refers to the amount of time necessary for a nominal dose of an active pharmaceutical ingredient (API) to attain maximum blood plasma concentration after administration with a high efficiency nebulizer.

[Tₘₐₓ^{Conv}] as used herein, refers to the amount of time necessary for a nominal dose of an active pharmaceutical ingredient (API) to attain maximum blood plasma concentration after administration with a conventional nebulizer.

The term "treat" and its grammatical variants (e.g. "to treat," "treating," and "treatment") refer to administration of an active pharmaceutical ingredient to a patient with the purpose of ameliorating or reducing the incidence of one or more symptoms of a condition or disease state in the patient. Such symptoms may be chronic or acute; and such amelioration may be partial or complete. In the present context, treatment entails administering a muscarinic antagonist (optionally in combination with a beta 2-adrenoreceptor agonist) to a patient via a pulmonary inhalation route.

The term "prophylaxis" refers to administration of an active pharmaceutical ingredient to a patient with the purpose of reducing the occurrence or recurrence of one or more acute symptoms associated with a disease state in the patient. In the present context, prophylaxis entails administering a muscarinic antagonist (optionally in combination with a beta 2-adrenoreceptor agonist) to a patient via a pulmonary inhalation route. Thus, prophylaxis includes reduction in the occurrence or recurrence rate of acute exacerbations in chronic obstructive pulmonary disease (COPD). However, prophylaxis is not intended to include complete prevention of onset of a disease state in a patient who has not previously been identified as suffering from a pulmonary condition or disease; nor does prophylaxis include prevention of pulmonary cancer.

As used herein, a difference is "significant" if a person skilled in the art would recognize that the difference is probably real. In some embodiments, significance may be determined statistically - in which case two measured parameters may be referred to as statistically significant. In some embodiments, statistical significance may be quantified in terms of a stated confidence interval (CI), e.g. greater than 90%, greater than 95%, greater than 98%, etc. In some embodiments, statistical significance may be quantified in terms of a p value, e.g. less than 0.5, less than 0.1, less than 0.05, etc. The person skilled in the art will recognize these expressions of significance and will know how to apply them appropriately to the specific parameters that are being compared.

In some embodiments described herein an active pharmaceutical ingredient (API) is a muscarinic antagonist. In some embodiments, the API is substantially free of other bronchodilating agents, such as beta-2 adrenoreceptor agonists, like formoterol, salmeterol and salbutamol (albuterol). In this context, "substantially free of other bronchodilating agents" indicates that the solution contains no other bronchodilating agent or contains less than a quantity of another bronchodilating agent that would be sufficient to materially affect the properties of the muscarinic antagonist solution. In some embodiments, the API is a muscarinic antagonist (optionally in combination with a beta 2-adrenoreceptor agonist). In some embodiments, the API is free of other bronchodilating agents, such as beta-2 adrenoreceptor agonists, like formoterol, salmeterol and salbutamol (albuterol). In this context, "free of other bronchodilating agents" means that the solution contains no other bronchodilating agent than the recited muscarinic antagonist, or contains less than a detectable amount of the other bronchodilating agents.

### Methods and Systems for the Treatment of Respiratory Conditions with HENs

The present invention provides methods and inhalation systems for treatment or prophylaxis of a respiratory condition in a patient, such as chronic obstructive pulmonary disease (COPD), and optionally chronic bronchitis and/or emphysema. In some embodiments, the methods and inhalation systems comprise administering to a patient a nominal dose of an active pharmaceutical ingredient (API), e.g. muscarinic antagonist (optionally in combination with a beta 2-adrenoreceptor agonist) in an aqueous inhalation solution with a high efficiency nebulizer, wherein delivering the nominal dose of the muscarinic antagonist to the patient provides one or more of the following advantages: (1) an enhanced pharmacokinetic profile as compared to administration with a conventional nebulizer; (2) an enhanced therapeutic effect as compared to administration with a conventional nebulizer; (4) an enhanced lung deposition (deposited lung dose) evidenced by scintigraphy or deconvolution, or derived from suitable in vitro indicators such as enhanced RDDR, RF, GSD, and/or a MMAD values as compared to administration with a conventional nebulizer; (5) reduced administration times, periods, and/or volumes as compared to administration with a conventional nebulizer; (6) a reduction in adverse side effects associated with API treatment and optionally a longer duration of therapeutic effect as compared to administration with a conventional nebulizer; (7) optional administration with a Beta 2-Adrenoreceptor Agonist and optionally a corticosteroid; and (8) an enhanced method of treatment of acute exacerbations of a respiratory condition in a patient, e.g. COPD. In some embodiments, the methods and inhalation systems comprise administering to a patient a nominal dose of an increased concentration of API in an aqueous inhalation device, metered dose inhaler (MDI), conventional nebulizer, or high efficiency nebulizer.

### Inhalation Therapy

An inhalation device, as used herein, refers to any device that is capable of administering a solution to the respiratory airways of a patient. Inhalation devices include conventional inhalation devices, such as metered dose inhalers (MDIs), conventional nebulizers, such as jet nebulizers, and high efficiency nebulizers, such as vibrating membrane nebulizers.

Inhalation nebulizers, or atomizers, are also commonly used for the treatment of respiratory diseases. Inhalation nebulizers deliver therapeutically effective amounts of pharmaceuticals by forming an aerosol which includes droplet sizes that can easily be inhaled. The aerosol can be used, for example, by a patient within the bounds of an inhalation therapy, whereby the therapeutically effective pharmaceutical or drug reaches the patient's respiratory tract upon inhalation.

### High Efficiency Nebulizers

High efficiency nebulizers are inhalation devices that comprise a microperforated membrane through which a liquid solution is converted through electrical or mechanical means into aerosol droplets suitable for inhalation. High efficiency nebulizers can deliver a large fraction of a loaded dose to a patient. In some embodiments, the high efficiency nebulizer also utilizes one or more actively or passively vibrating microperforated membranes. In some embodiments, the high efficiency nebulizer contains one or more oscillating membranes. In some embodiments, the high efficiency nebulizer contains a vibrating mesh or plate with multiple apertures and optionally a vibration generator with an aerosol mixing chamber. In some such embodiments, the mixing chamber functions to collect (or stage) the aerosol from the aerosol generator. In some embodiments, an inhalation valve is also used to allow an inflow of ambient air into the mixing chamber during an inhalation phase and is closed to prevent escape of the aerosol from the mixing chamber during an exhalation phase. In some such embodiments, the exhalation valve is arranged at a mouthpiece which is removably mounted at the mixing chamber and through which the patient inhales the aerosol from the mixing chamber. Still yet, in some embodiments, the high efficiency nebulizer contains a pulsating membrane. In some embodiments, the high efficiency nebulizer is continuously operating.

In some embodiments, the high efficiency nebulizer contains a vibrating microperforated membrane of tapered nozzles that generates a plume of droplets without the need for compressed gas. In these embodiments, a solution in the microperforated membrane nebulizer is in contact with a membrane, the opposite side of which is open to the air. The membrane is perforated by a large number of nozzle orifices of an atomizing head. An aerosol is created when alternating acoustic pressure in the solution is built up in the vicinity of the membrane causing the fluid on the liquid side of the membrane to be emitted through the nozzles as uniformly sized droplets.

Some embodiments of high efficiency nebulizers use passive nozzle membranes and a separate piezoelectric transducer that stimulates the membrane. In contrast, some high efficiency nebulizers employ an active nozzle membrane, which use the acoustic pressure in the nebulizer to generate very fine droplets of solution via the high frequency vibration of the nozzle membrane.

Some high efficiency nebulizers contain a resonant system. In some such high efficiency nebulizers, the membrane is driven by a frequency for which the amplitude of the vibrational movement at the center of the membrane is particularly large, resulting in a focused acoustic pressure in the vicinity of the nozzle; the resonant frequency may be about 100 kHz. A flexible mounting is used to keep unwanted loss of vibrational energy to the mechanical surroundings of the atomizing head to a minimum. In some embodiments, the vibrating membrane of the high efficiency nebulizer may be made of a nickel-palladium alloy by electroforming.

In some embodiments, the high efficiency nebulizer achieves lung deposition (deposited lung dose) of at least about 30%, at least about 35%, at least about 40%, at least about 45%, at least about 50%, at least about 55%, at least about 60%, about 30% to about 60%, about 30% to about 55%, about 30% to about 50%, about 30% to about 40%, about 30% to about 90%, about 40% to about 80%, about 50% to about 60%, or about 60% to about 70% based on the nominal dose of the muscarinic antagonist (optionally in combination with a beta 2-adrenoreceptor agonist) administered to the patient. In some embodiments, the high efficiency nebulizer achieves a respirable dose delivery rate (RDDR) of at least about 2 times, at least about 3 times or at least about 4 times the RDDR achievable with a conventional nebulizer. In some embodiments where the muscarinic antagonist is glycopyrrolate is the RDDR is at least about 100 µg/min, at least about 150 µg/min, at least about 200 µg/min, about 100 µg/min to at least about 5,000 µg/min, about 150 µg/min to about 4,000 µg/min or about 200 µg/min to about 3,500 µg/min. In some embodiments, wherein the muscarinic antagonist is glycopyrrolate, the high efficiency nebulizer achieves an output rate of at least about 120 µg/min, at least about 150 µg/min, at least about 200 µg/min or at least about 200 µg/min to at least about 5,000 µg/min. In some embodiments, the high efficiency nebulizer provides a respirable fraction (RF) of muscarinic antagonist of at least about 60%, at least about 65%, at least about 70%, at least about 75%, at least about 80%, at least about 85%, at least about 90%, about 60% to about 95%, about 65% to about 95%, about 65% to about 90% or about 70% to about 90%. In some embodiments, the high efficiency nebulizer provides a Geometric Standard Deviation (GSD) of emitted droplet size distribution of the solution administered with a high efficiency nebulizer of about 1.1 to about 2.1, about 1.2 to about 2.0, about 1.3 to about 1.9, at least about 1.4 to about 1.8, at least about 1.5 to about 1.7, about 1.4, about 1.5, or about 1.6. In some embodiments, administration of the muscarinic antagonist with the high efficiency nebulizer provides a Mass Median Aerodynamic Diameter (MMAD) of droplet size of the solution emitted with the high efficiency nebulizer of about 1 µm to about 5 µm, about 2 to about 4 µm, about 3 µm to about 4 µm, about 3 to about 4 µm, or about 3.5 to about 4.0 µm. In some particular embodiments, the high efficiency nebulizer provides droplets having a particular combination of MMAD and GSD, for example: an MMAD of less than about 5 µm and a GSD of about 1.1 to about 2.1; an MMAD of less than about 4.5 µm and a GSD of about 1.1 to about 2.1; an MMAD of about 1 µm to about 5 µm and a GSD of about 1.1 to about 2.1; an MMAD of about 1.5 to about 4.5 µm and a GSD of about 1.1 to about 2.1; an MMAD of less than about 5 µm and a GSD of about 1.1 to about 2.0; an MMAD of less than about 4.5 µm and a GSD of about 1.1 to about 2.0; an MMAD of about 1 µm to about 5 µm and a GSD of about 1.1 to about 2.0; an MMAD of about 1.5 to about 4.5 µm and a GSD of about 1.1 to about 2.0; an MMAD of less than about 5 µm and a GSD of about 1.1 to about 1.9; an MMAD of less than about 4.5 µm and a GSD of about 1.1 to about 1.9; an MMAD of about 1 µm to about 5 µm and a GSD of about 1.1 to about 1.9; an MMAD of about 1.5 to about 4.5 µm and a GSD of about 1.1 to about 1.9; an MMAD of less than about 5 µm and a GSD of about 1.1 to about 1.8; an MMAD of less than about 4.5 µm and a GSD of about 1.1 to about 1.8; an MMAD of about 1 µm to about 5 µm and a GSD of about 1.1 to about 1.8; or an MMAD of about 1.5 to about 4.5 µm and a GSD of about 1.1 to about 1.8.

In some embodiments, the high efficiency nebulizer provides muscarinic antagonist lung deposition (deposited lung dose) of at least about 15%, at least about 20%, at least about 25%, at least about 30%, at least about 35%, at least about 40%, at least about 45%, at least about 50%, at least about 55%, at least about 60%, about 20% to about 40%, about 25% to about 35%, about 25 to about 30%, about 35% to about 90%, about 40% to about 80%, about 50% to about 60%, or about 60% to about 70% based on the nominal dose of the muscarinic antagonist. In some embodiments, the high efficiency nebulizer provides for one or more of (a) or (b); and one or more of (i), (ii), (iii) or (iv): (a) a respirable dose delivery rate (RDDR) of at least about 2 times, 3 times or 4 times the RDDR achievable with a conventional nebulizer (for example, where the muscarinic antagonist is glycopyrrolate, in some embodiments, RDDR is at least about 100 µg/min, at least about 150 µg/min, at least about 200 µg/min, about 100 µg/min to about 5,000 µg/min, about 150 µg/min to about 4,000 µg/min or about 200 µg/min to about 3,500 µg/min); (b) an output rate of muscarinic antagonist of at least about 2 times, 3 times or 4 times the output rate achievable with a conventional nebulizer (for example, where the muscarinic antagonist is glycopyrrolate, the output rate is at least about 120 µg/min, at least about 150 µg/min, at least about 200 µg/min or at least about 200 µg/min to about 5,000 µg/min); (i) a respirable fraction (RF) of muscarinic antagonist of at least about 30 %, at least about 35%, at least about 40%, at least about 45%, at least about 50%, at least about 55%, at least about 65% to at least about 75% or at least about 75% to at least about 85% respirable fraction upon administration; (ii) a Geometric Standard Deviation (GSD) of emitted droplet size distribution of the solution administered with a inhalation device of about (iii) about 1.1 to about 2.1 , about 1.2 to about 2.0, about 1.3 to about 1.9, about 1.4 to about 1.8, about 1.5 to about 1.7, about 1.4, about 1.5, or about 1.6; (iv) or a Mass Median Aerodynamic Diameter (MMAD) of droplet size of the solution emitted with the inhalation device of about 1 µm to about 5 µm, about 2 to about 4 µm, about 3 to about 4 µm, or about 3.5 to about 4.0 µm.

In accordance with the invention, in some embodiments, a high efficiency nebulizer may be adapted or adaptable to operate in conjunction with a unit dosage form, such as an ampule or vial, which contains a single dose of a muscarinic antagonist composition for the treatment of COPD. The unit dosage form comprises a container that contains an inhalation solution comprising the muscarinic antagonist, such as glycopyrrolate. The container is adapted to cooperate with the high efficiency nebulizer device in such a way as to permit administration of the nominal dose of the inhalation solution to a patient. In some embodiments, the high efficiency nebulizer and the unit dosage form are configured so that they are useable together, but not with other devices or dosage forms. In some particular embodiments, the unit dosage form is configured such that it fits into a keyhole-like structure in the high efficiency nebulizer, but will not operate with other nebulizer devices. In such embodiments, the high efficiency nebulizer is configured such that it will accept and properly operate with the unit dosage form containing the muscarinic antagonist, but not with other dosage forms.

Additional features of a high efficiency nebulizer with perforated membranes are disclosed in U.S. Pat. Nos. 6,962,151, 5,152,456, 5,261,601, and 5,518,179, each of which is hereby incorporated by reference in its entirety. Other embodiments of the high efficiency nebulizer contain oscillatable membranes. Features of these high efficiency nebulizers are disclosed in 7,252,085; 7,059, 320; 6,983,747, each of which is hereby incorporated by reference in its entirety.

Commercial high efficiency nebulizers are available from: PARI (Germany) under the trade name eFlow®; Aerogen, Ltd.(Ireland) under the trade names AeroNeb® Go and AeroNeb® Pro, AeroNeb® Solo, and other nebulizers utilizing the OnQ® nebulizer technology; Respironics (Murrysville, CA) under the trade names I-Neb®; Omron (Bannockburn, IL) under the trade name Micro-Air®; Activaero (Germany) under the trade name Akita®, and AerovectRx (Atlanta, GA) under the trade name AerovectRx®.

### Conventional Nebulizers

Conventional nebulizers include, for example jet nebulizers or ultrasonic nebulizers. Jet nebulizers generally utilize compressors to generate compressed air, which breaks the liquid medication into small breathable droplets, which form an aerosolized (atomized) mist. In some of these embodiments, when the patient breathes in, a valve at the top opens, which then allows air into the apparatus, thereby speeding up the mist generation; when the patient breathes out, the top valve closes, thereby slowing down the mist generation while simultaneously permitting the patient to breathe out through the opening of a mouthpiece flap.

In general, conventional nebulizers are characterized by relatively low efficiency in delivery of a muscarinic antagonist to the lung. Thus, a conventional nebulizer, such as a jet nebulizer, is characterized by one or more of the following: (1) about 25% or about 20% or less calculated respirable dose or measured deposited lung dose as a percentage of the nominal dose of API administered to the patient; (2) a respirable dose delivery rate (RDDR) of less than about 100 µg/min of the muscarinic antagonist administered to the patient; (3) an output rate of less than about 100 µg/min API administered to the patient; (4) a residual volume of greater than about 10% of the nominal dose of the muscarinic antagonist.

Some conventional nebulizers are disclosed in U.S. Patent Nos. 6,513,727, 6,513,519, 6,176,237, 6,085,741, 6,000,394, 5,957,389, 5,740,966, 5,549,102, 5,461,695, 5,458,136, 5,312,046, 5,309,900, 5,280,784, and 4,496,086, each of which is hereby incorporated by reference in its entirety.

Commercial conventional nebulizers are available from: PARI (Germany) under the trade names PARI LC Plus®, LC Star®, and PARI-Jet®; A & H Products, Inc. (Tulsa, OK) under the trade name AquaTower®; Hudson RCI (Temecula, CA) under the trade name AVA-NEB®; Intersurgical, Inc. (Liverpool, NY) under the trade name Cirrus®; Salter Labs (Arvin, CA) under the trade name Salter 8900®; Respironics (Murrysville, PA) under the trade name Sidestream®; Bunnell (Salt Lake City, UT) under the trade name Whisper Jet®; Smiths-Medical (Hyth Kent, UK) under the trade name Downdraft®, and DeVilbiss (Somerset, PA) under the trade name DeVilbiss®.

### Active Ingredient(s)

### Muscarinic Antagonists

Acetylcholine released from cholinergic neurons in the peripheral and central nervous systems affects many different biological processes through interaction with two major classes of acetylcholine receptors: the nicotinic and the muscarinic receptors.

Muscarinic acetylcholine receptors are widely distributed in vertebrate organs where they mediate many vital functions. Three subtypes of muscarinic acetylcholine receptors have been identified as important in the lung, M1, M2, and M3, each with its unique pharmacological properties and a product of a distinct gene. These three subtypes are also located in organs other than the lung.

In the lung, M3 muscarinic receptors mediate smooth muscle contraction. Stimulation of M3 muscarinic receptors activate the enzyme phospholipase C via binding of the stimulatory G protein Gq/11 (Gs), leading to liberation of phosphatidyl inositol-4, 5-bisphosphate, resulting in phosphorylation of contractile proteins and bronchial constriction. M3 muscarinic receptors are also found on pulmonary submucosal glands. Stimulation of this population of M3 muscarinic receptors results in mucus secretion. M2 muscarinic receptors make up approximately 50-80% of the cholinergic receptor population on airway smooth muscles. Under normal physiological conditions, M2 muscarinic receptors provide tight control of acetylcholine release from parasympathetic nerves. M1 muscarinic receptors are found in the pulmonary parasympathetic ganglia where they function to enhance neurotransmission.

Muscarinic acetylcholine receptor dysfunction in the lungs has been noted in a variety of different pathophysiological states. In asthma and COPD patients, inflammatory conditions lead to loss of inhibitory M2 and M3 muscarinic acetylcholine autoreceptor function on parasympathetic nerves supplying the pulmonary smooth muscle, causing an increased release of acetylcholine. This dysfunction in muscarinic receptors results in airway hyperreactivity and hyperresponsiveness.

Muscarinic acetylcholine receptor antagonist agents, or muscarinic antagonists, have the ability to inhibit the action of the neurotransmitter acetylcholine by blocking its interaction with muscarinic cholinergic receptors in general, and its interaction with specific muscarinic receptor subtypes in particular. Muscarinic antagonists thereby prevent the effects resulting from the passage of unnecessary impulses through the parasympathetic nerves mediated by increased stimulation in patients with dysfunctional receptors, resulting in, among other physiological effects, relaxation of smooth muscles in the lung.

Aclidinium, ((3R-3-{[hydroxydi(thiophen-2-yl)acetyl]oxy}-1-(3-phenoxypropyl)-1-azoniabicyclo[2.2.2]octane bromide), is a specific long-acting muscarinic receptor antagonist. Aclidinium is in development for use as an anticholinergic agent. Clinically, aclidinium has been tested in a dry powder inhaled format.

In some embodiments of the present invention, the muscarinic antagonist is aclidinium and is administered at a nominal dosage of 100 µg/dose to about 5 mg/dose, about 50 µg/dose to about 2 mg/dose or about 50 µg/dose to about 1 mg per dose. In other embodiments, aclidinium is given in 100 µg, 200 µg, 300 µg, 400 µg, 500 µg, 600 µg, 700 µg, 800 µg, 900 µg, or 1,000 µg doses.

The process of making aclidinium is known by a person of ordinary skill in the art. Aclidinium can be made by a number of known methods including those described in U.S. Patent 6,750,226, which is incorporated herein by reference in its entirety, and which sets forth several structurally related muscarinic antagonists. Additional examples of muscarinic antagonists are set forth in US Patent Nos. 7,312,231 and 7,208,501, each of which is incorporated herein by reference in its entirety.

Trospium (endo-3-[(Hydroxydiphenylacetyl)oxy]spiro[8-azoniabicyclo[3.2.1]ocatane-8,1'-pyrrolidinium] chloride benzilate), is a specific long-acting muscarinic receptor antagonist. Trospium has been known for many years to be an effective anticholinergic agent. Clinically, trospium has been used in several indications and been delivered by a number of different routes. Currently, trospium is used as a urinary antispasmotic and is sold under the brand name Sanctura®.

In some embodiments of the present invention, the muscarinic antagonist is trospium and is administered at a nominal dosage of 10 µg/dose to about 5 mg/dose, about 10 µg/dose to about 2 mg/dose or about 50 µg/dose to about 1 mg per dose. In other embodiments, trospium is given in 10 µg, 50 µg, 100 µg, 200 µg, 300 µg, 400 µg, 500 µg, 600 µg, 700 µg, 800 µg, 900 µg, or 1,000 µg doses.

The process of making trospium is known by a person of ordinary skill in the art. Trospium can be made by a number of known methods including those described in U.S. Patent 3,480,626, which is incorporated herein by reference in its entirety.

Glycopyrrolate, 3-[(cyclopentylhydroxyphenylacetyl)oxy]-1,1-dimethylpyrrolidinium, is a specific long-acting muscarinic receptor antagonist. Glycopyrrolate has been known for many years to be an effective anticholinergic agent. Clinically, glycopyrrolate has been used in several indications and been delivered by a number of different routes. Currently, glycopyrrolate is approved for use as an injectable compound to reduce secretions during anesthesia and also as an oral product for treating gastric ulcers.

In some embodiments of the present invention, the muscarinic antagonist is glycopyrrolate and is administered with a high efficiency nebulizer at a nominal dosage of about 50 µg/dose to about 1 mg/dose, about 50 µg/dose to about 0.5 mg/dose, about 50 µg/dose to about 250 µg/dose, about 50 µg/dose to about 150 µg/dose, about 50 µg/dose to about 125 µg/dose, about 50 µg/dose to about 100 µg/dose, less than about 150 µg/dose, less than about 100 µg/dose, equal to or less than about 80 µg/dose, 100 µg/dose to about 5 mg/dose, about 200 µg/dose to about 2 mg/dose or about 250 µg/dose to about 1 mg per dose.

The process of making glycopyrrolate is known by a person of ordinary skill in the art. Glycopyrrolate can be made as follows. First, alpha-phenylcyclopentaneglycolic acid is esterified by refluxing with methanol in the presence of hydrochloric acid and the resulting ester is transesterified with 1-methyl-3-pyrrolidinol using sodium as a catalyst; the transester is then reacted with methyl bromide to give glycopyrrolate. U.S. Pat No. 6,433,003, which describes this process in more detail, is hereby incorporated by reference in its entirety.

Glycopyrrolate for injectable and oral administration is readily commercially available. Injectable glycopyrrolate in commercial administrations are sold by: Baxter Healthcare, Inc. (Deerfiled, IL) under the trade name Robinul and by Luitpold Pharmaceuticals, Inc. (Shirley, NY) under the generic name glycopyrrolate. Oral glycopyrrolate is commercially available under the generic name glycopyrrolate from Corepharma, LLC (Middlesex, NJ) and Kali Laboratories, Inc. (Somerset, NJ), and is available from Sciele Pharma, Inc. (Atlanta, GA) under the trade names Robinul and Robinul Forte.

Tiotropium is another long-acting muscarinic antagonist that may be mentioned. In some embodiments, the dose of tiotropium administered with a high efficiency nebulizer will be less than about 15 µg, less than about 12 µg, less than about 10 µg or less than about 8 µg. In some embodiments, the dose will be in a range of about 1-10 µg, about 1-9 µg, about 1-8 µg or about 1-5 µg, about 1 µg, about 2 µg, about 3 µg, about 4 µg, about 5 µg, about 6 µg, about 7 µg, about 8 µg, about 9 µg or about 10 jug per dose.

Muscarinic antagonists can be long-acting or short-acting. Long-acting muscarinic antagonists have a therapeutic effect lasting greater than about 6 hours. Short-acting muscarinic antagonists have a duration of therapeutic effect of less than about 6 hours. Long-acting muscarinic antagonists include, but are not limited to, glycopyrrolate, tiotropium, aclidinium, trospium, QAT370, GSK233705, GSK656398, BEA 2180, or a pharmaceutical acceptable derivative, salt, enantiomer, diastereomer, or racemic mixtures thereof.

Short-acting muscarinic antagonists include, but are not limited to ipratropium, oxitropium or a pharmaceutical acceptable derivative, salt, enantiomer, diastereomer, or racemic mixtures thereof.

In some embodiments, the muscarinic antagonist is glycopyrrolate, tiotropium, aclidinium, trospium, QAT370, GSK233705, GSK 656398, BEA2180, ipratropium, oxitropium, oxybutynin or a pharmaceutical acceptable derivative, salt, enantiomer, diastereomer, or a pharmaceutical acceptable derivative, salt, enantiomer, diastereomer, or racemic mixture thereof.

### Beta 2-Adrenoreceptor Agonists

The stimulation of beta 2-adrenergic receptors stimulate adenylate cyclase, resulting in an increased level of the second messenger cAMP that in turn leads to decreased intracellular calcium concentration and consequently smooth muscle relaxation. Stimulation of certain beta 2-adrenoreceptor receptors in particular causes hydrolysis of polyphosphoinositides and mobilization of intracellular calcium which results in a variety of calcium mediated responses such as smooth muscle contraction. Consequently, inhibition of this receptor activation prevents the intracellular calcium increase and leads to smooth muscle relaxation.

Beta 2-adrenoreceptor agonists can be long-acting or short-acting. Long-acting beta 2-adrenoreceptor agonists (LABAs) have a therapeutic effect lasting greater than about 6 hours. Short-acting beta 2-adrenoreceptor agonists (SABAs) have a duration of therapeutic effect of less than about 6 hours.

Compounds having beta 2 -adrenoreceptor agonist activity with a long-acting or short-acting effect have been developed to treat respiratory conditions. Such compounds include, but are not limited to, albuterol; bambuterol; bitolterol; broxaterol; carbuterol; clenbuterol; ibuterol; sulfonterol; isoproterenol; trimetoquinol; formoterol; desformoterol; hexoprenaline; ibuterol; indacaterol; isoetharine; isoprenaline; isoproterenol; levalbuterol; metaproterenol; picumeterol; pirbuterol; procaterol; reproterol; rimiterol; salbutamol; salmeterol; sulfonterol; terbutaline; trimetoquinol; tulobuterol; and TA-2005 (8-hydroxy-5-((1R)-1-hydroxy-2-(N-((1R)-2-(4-µmethoxyphenyl)-1-methylethyl) amino)ethyl)-carbostyril hydrochloride); or a or a pharmaceutical acceptable derivative, salt, enantiomer, diasteriomer, or racemic mixtures thereof.

Formoterol is a long-acting beta 2-adrenoreceptor compound. The process of making formoterol is known by one of skill in the art. Formoterol is derived from adrenaline and is used as a beta 2-adrenoreceptor agonist in inhalation therapy of respiratory diseases. Formoterol has been formulated as a dry powder and administered via devices such as the Turbuhaler® and the Aerolizer®.

Formoterol is also available as a tablet and a dry syrup in certain areas of the world (e.g., Atock®, marketed by Yamanouchi Pharmaceutical Co. Ltd., Japan). Formoterol administrations are also available in other areas (e.g., Europe and U.S.) for propellant-based metered dose inhalers and dry powder inhalers (e.g., Turbuhaler®, Aerolizer.® and Foradil Aerolizer®). None of these administrations are water based solutions.

Commercial administrations of arformoterol tartrate (formoterol) are sold by Sepracor, Inc. (Marlborough, MA) under the trade name Brovana®. Formoterol fumarate is sold by several companies including AstraZeneca, Inc. (London, England) under the trade name Symbicort®, Novartis International AG (Basel, Switzerland) under the trade names Foradil® and Certihaler®, and Dey, L.P. (Napa, CA) under the trade name Perforomist®.

Salmeterol is a long-acting beta 2-adrenoreceptor compound. The process for making salmeterol is known by a person of ordinary skill in the art and is described in U.S. Pat. No. 4,992,474, which is hereby incorporated by reference. Commercial administrations of salmeterol are sold by GlaxoSmithKline, Inc. (Triangle Park, NC) under the trade names Advair® and Serevent®.

In some embodiments, the inhalation solution comprising the Muscarinic Antagonist(s) further comprises a corticosteroid, such as fluticasone, mometasone, beclomethasone, triamcinolone, fluniolide, ciclesonide, or budesonide. In some embodiments, the inhalation solution further comprises an excipient, including an organic acid, such as citric acid, ascorbic acid or optionally a combination of both, pilocarpine, cevimeline or carboxymethylcellulose, or a mucolytic compound. In some embodiments, the inhalation solution contains muscarinic antagonist (such as glycopyrrolate) a beta 2-adrenoreceptor agonist and a corticosteroid.

### Inhalation Solutions

The present invention relates to methods and inhalation systems for the use of inhalation solutions in an inhalation device for the treatment or prophylaxis of a respiratory condition in a patient, such as COPD, chronic bronchitis, or emphysema. In some embodiments, the methods and inhalation systems comprise administering to the patient a nominal dose of one or more API, for example muscarinic antagonist (optionally in combination with a beta 2-adrenoreceptor agonist and/or a corticosteroid) in an aqueous inhalation solution with an inhalation device, e.g. a high efficiency nebulizer, conventional nebulizer, and optionally a conventional inhalation device.

In some embodiments, the methods and inhalation systems employ an aqueous inhalation solution containing glycopyrrolate (optionally in combination with a beta 2-adrenoreceptor agonist and/or a corticosteroid) is administered with a high efficiency nebulizer at a concentration of at least about 0.5 mg/mL. In some embodiments, an aqueous inhalation solution containing glycopyrrolate is administered with a high efficiency nebulizer at a concentration greater than about 0.5 mg/mL. In some embodiments, an aqueous inhalation solution containing glycopyrrolate is administered with a high efficiency nebulizer at a concentration of 0.05 mg/mL to about 2.0 mg/mL, at least about 0.05 mg/mL, at least about 0.5 mg/mL, at least about 1.0 mg/mL to about 2.0 mg/mL, at least about 0.25 mg/mL, at least about 0.5 mg/mL, at least about 1.0 mg/mL, at least about 1.5 mg/mL, or at least about 2.0 mg/mL. Without wishing to be bound by theory, it is believed that the higher concentration of muscarinic antagonist, for example glycopyrrolate, results in saturation and/or targeting of a greater number of muscarinic receptors with the muscarinic antagonist and/or targeting of the muscarinic receptors with the muscarinic antagonist for a greater period of time. In some embodiments, it is believed that administration of a muscarinic antagonist, such as glycopyrrolate, at a concentration of at least about 0.5 mg/mL will result in greater duration and/or magnitude of therapeutic effect. Without wishing to be bound by theory, it is believed that the higher concentration of muscarinic antagonist, for example glycopyrrolate, coupled with the improved delivery and/or lung distribution achievable with a high efficiency nebulizer, results in saturation and/or targeting of a greater number of muscarinic receptors with the muscarinic antagonist and/or targeting of the muscarinic receptors with the muscarinic antagonist for a greater period of time. In some embodiments, it is believed that administration of a muscarinic antagonist, such as glycopyrrolate, in a nebulized form having an MMAD of less than about 4 µm and a GSD of less than about 1.8, wherein the loaded dose has a concentration of at least about 0.5 mg/mL, will result in greater duration and/or magnitude of therapeutic effect. Without wishing to be bound by theory, it is believed that this combination of characteristics results in saturation and/or targeting of a greater number of muscarinic receptors with the muscarinic antagonist and/or targeting of the muscarinic receptors for a greater period of time.

In some embodiments, the methods, devices and systems employ an aqueous inhalation solution containing glycopyrrolate, at an administration of about 100 µg/dose to about 5 mg/dose, about 200 µg/dose to about 2 mg/dose, or about 250 µg/dose to about 1 mg per dose. In some embodiments, the methods, devices and systems comprise a composition containing glycopyrrolate in an amount of about 50 µg/dose to about 1 mg/dose, about 50 µg/dose to about 0.5 mg/dose, about 50 µg/dose to about 250 µg/dose, about 50 µg/dose to about 150 µg/dose, about 50 µg/dose to about 125 µg/dose, about 50 µg/dose to about 100 µg/dose, less than about 150 µg/dose, less than about 100 µg/dose, equal to or less than about 80 µg/dose, 100 µg/dose to about 5 mg/dose, about 200 µg/dose to about 2 mg/dose or about 250 µg/dose to about 1 mg per dose.

In some embodiments, the aqueous inhalation solution is administered with an inhalation device, e.g. high efficiency nebulizer, at a fill volume of less than about 0.5 mL, at least about 0.5 mL to about 1.5 mL, at least about 0.25 mL or less, at least about 0.5 mL to about 1.5 mL, at least about 1.5 mL, or at least about 2.0 mL. In some embodiments, the aqueous inhalation solution is administered with an inhalation device, e.g. high efficiency nebulizer, at a fill volume of at least about 0.25 mL to about 2.0 mL, about 0.5 mL to about 1.5 mL, about 0.5 mL to about 1.0 mL, about 0.5 mL or less, about 1 mL or less, about 1.5 mL or less, or about 2.0 mL or less. In some embodiments, the aqueous inhalation solution is administered with an inhalation device, e.g. high efficiency nebulizer, which provides for a residual volume of muscarinic antagonist after administration of the muscarinic antagonist of less than about 10%, less than about 5%, or less than about 3% of the nominal dose.

In some embodiments, the aqueous inhalation solution is administered in about 0.25 to about 10 minutes, about 0.50 to about 8 minutes, less than about 8, less than about 7, less than about 6, less than about 5, less than about 4, less than about 3, less than about 2, less than about 1.75 minutes, or less than about 1.5 minutes. In some embodiments, the aqueous inhalation solution is administered in about 3 minutes or less.

In some embodiments, a muscarinic antagonist is co-administered with a beta 2-adrenoreceptor agonist in the aqueous inhalation solution. In some embodiments, the beta 2-adrenoreceptor agonist is a long-acting beta agonist (LABA). In some embodiments, the beta 2-adrenoreceptor agonist is a short-acting beta agonist (SABA). In some embodiments, the beta 2-adrenoreceptor agonist is either a SABA or a LAMA.

In some embodiments, the nominal dose administered with the high efficiency nebulizer is a muscarinic antagonist (optionally in combination with a beta 2-adrenoreceptor agonist) that is substantially free of preservative, such as benzyl alcohol. In some embodiments, the nominal dose of muscarinic antagonist (optionally in combination with a beta 2-adrenoreceptor agonist) is in an inhalation solution that further comprises at least one excipient or active pharmaceutical ingredient. In some embodiments, the excipient or active ingredient is a member of the group consisting of organic acid (such as a low molecular weight organic acid like citric acid or ascorbic acid), an antioxidant (such as EDTA), an osmolarity adjusting agent (such as a salt like sodium chloride) or a pH buffer.

In some embodiments, the inhalation solution comprising the muscarinic antagonist(s) further comprises a corticosteroid, such as fluticasone, mometasone, beclomethasone, triamcinolone, fluniolide, ciclesonide, or budesonide. In some embodiments, the inhalation solution further comprises an excipient or an active pharmaceutical ingredient. In some embodiments, the excipient or active pharmaceutical ingredient is an organic acid (such as citric acid, ascorbic acid, or both), pilocarpine, cevimeline, carboxymethylcellulose, or a mucolytic compound.

### Characterization of Inhalation Devices

The efficiency of a particular inhalation device can be measured by many different ways, including an analysis of pharmacokinetic properties, measurement of lung deposition (deposited lung dose) percentage, measurement of respirable dose delivery rates (RDDR), a determination of output rates, respirable fraction (RF), geometric standard deviation values (GSD), and mass median aerodynamic diameter values (MMAD) among others.

A person skilled in the art is knowledgeable of methods and systems for examining a particular inhalation device. One such system consists of a computer and a hollow cylinder in a pump with a connecting piece to which an inhalation device is to be connected. In the pump there is a piston rod, which extends out of the hollow cylinder. A linear drive unit can be activated in such a manner that one or more breathing pattern will be simulated on the connecting piece of the pump. In order to be able to carry out the evaluation of the inhalation device, the computer is connected in an advantageous configuration with a data transmission. With the aid of the data transmission, the computer can be connected with another computer with specific data banks, in order to exchange the data of breathing patterns. In this manner, a breathing pattern library which is as representative as possible can be very rapidly formed. U.S. Pat. No. 6,106,479 discloses this method for examining an inhalation device in more detail, and is hereby incorporated by reference in its entirety.

### Pharmacokinetic Profile

Pharmacokinetics is concerned with the uptake, distribution, metabolism and excretion of a drug substance. A pharmacokinetic profile comprises one or more biological measurements designed to measure the absorption, distribution, metabolism and excretion of a drug substance. One way of visualizing a pharmacokinetic profile is by means of a blood plasma concentration curve, which is a graph depicting mean active ingredient blood plasma concentration on the Y-axis and time (usually in hours) on the X-axis. Some pharmacokinetic parameters that may be visualized by means of a blood plasma concentration curve include:
- AUCₗₐₛₜ: The area under the curve from time zero to time of last measurable concentration.
- AUC_{(0-∞)}: The total area under the curve.
- Cₘₐₓ: The maximum plasma concentration in a patient.
- Tₘₐₓ: The time to reach maximum plasma concentration in a patient

An enhanced pharmacokinetic profile in a patient can be indicated by increased AUCₗₐₛₜ, AUC_{(0-∞)}, Cₘₐₓ, or a decreased Tₘₐₓ. Enhanced levels of a pharmaceutical agent in the blood plasma of a patient may result in or more improved symptoms of an airway respiratory condition, e.g. COPD.

In some embodiments, a method or system described herein provides at least about a two-fold enhancement in pharmacokinetic profile, meaning that administration of an active pharmaceutical ingredient ("API"-e.g. a muscarinic antagonist, optionally in combination with a beta 2-adrenergic agonist) with a high efficiency nebulizer provides at least about a two-fold increase in one or more of AUCₗₐₛₜ, AUC_{(0-∞)}, or Cₘₐₓ as compared to the same or lower nominal dose of API administered with a conventional nebulizer.

In some embodiments, a method or system described herein provides at least about a two-fold enhancement in pharmacokinetic profile, meaning that administration of an active pharmaceutical ingredient ("API"-e.g. a muscarinic antagonist, optionally in combination with a beta 2-adrenergic agonist) with a high efficiency nebulizer provides at least about a 1.8-fold increase in one or more of AUCₗₐₛₜ, AUQ_{(0-∞)}, or Cₘₐₓ as compared to the same or lower nominal dose of API administered with a conventional nebulizer.

In some embodiments, a method or system described herein provides at least about a two-fold enhancement in pharmacokinetic profile, meaning that administration of an active pharmaceutical ingredient ("API"-e.g. a muscarinic antagonist, optionally in combination with a beta 2-adrenergic agonist) with a high efficiency nebulizer provides at least about a 1.5-fold increase in one or more of AUCₗₐₛₜ, AUC_{(0-∞)}, or Cₘₐₓ as compared to the same or lower nominal dose of API administered with a conventional nebulizer.

In some embodiments, a method or system described herein provides at least about a two-fold enhancement in pharmacokinetic profile, meaning that administration of an active pharmaceutical ingredient ("API"-e.g. a muscarinic antagonist, optionally in combination with a beta 2-adrenergic agonist) with a high efficiency nebulizer provides a comparable AUCₗₐₛₜ, AUC_{(0-∞)}, or Cₘₐₓ as compared to the same or lower nominal dose of API administered with a conventional nebulizer.

### Enhanced Therapeutic Effect

The assessment of therapeutic effect is known to those skilled in the art, such as pulmonologists trained to recognized the distinctions between various types of respiratory illnesses, including chronic obstructive airway disease ("COPD") and asthma. Assessment of efficacy may be carried out by various methods known to the person skilled in the art, and may include both objective and subjective (patient-generated) measures of efficacy. Objective measures of efficacy can be obtained inter alia by spirometry; and subjective measures of efficacy can be obtained for example by employing one or more patient symptom questionnaires or surveys. In some embodiments, the methods and systems herein are for treatment of COPD, and thus such embodiments are discussed in further detail below. It is considered that embodiments of the methods and symptoms described herein (including those employing administration of muscarinic antagonist, optionally in combination with a beta-2 adrenoreceptor agonist and/or a corticosteroid, with a high efficiency nebulizer or at a high concentration) will provide superior efficacy in treatment of COPD as compared to treatment with conventional methods (such as those in which muscarinic antagonist is administered with a conventional nebulizer and/or at a lower concentration).

### COPD Efficacy Assessment

COPD is a progressive, chronic disease of the airways, characterized by chronic inflammation and destruction of the airways and lung parenchyma, resulting in airflow obstruction. Thus, efficacy in the treatment of COPD refers to the ability to restore airflow to the patient. In some cases, especially in older and immune-compromised patients, COPD can be further characterized by exacerbations - acute, often pathogen- or allergen-induced, degradation of airflow. There are several indicators (endpoints) of efficacy in the treatment of COPD. Some efficacy endpoints that are used in COPD studies are set forth below. It is considered that a muscarinic antagonist will demonstrate efficacy in one or more of these tests. In particular, it is considered that in some embodiments a nominal dose of a muscarinic antagonist administered with a high efficiency nebulizer will out-perform substantially the same or lower nominal dose of muscarinic antagonist administered with a conventional nebulizer, as determined by one or more of these endpoints.

**Pulmonary function tests:** Pulmonary function testing by spirometry is a useful way to assess airflow obstruction and, therefore, is a useful way to assess the efficacy of COPD treatment as well as to compare the relative merits of different COPD treatments-e.g. administration of different dosages of active pharmaceutical ingredient ("API"), administration of substantially the same dosages of API with different delivery devices, administration of or administration of different dosages of API with different delivery devices. Forced expiratory volume in one second (FEV₁) obtained from typical spirometry is commonly used as an efficacy endpoint because FEV₁ is a reflection of the extent of airway obstruction. Spirometry is also well-standardized, is easy to perform and provides consistent, reproducible results across different pulmonary function laboratories. Air-trapping and hyperinflation are common features in COPD, particularly in emphysematous-type, and are reflected in parameters of lung function testing, such as an elevation in the residual volume to total lung capacity ratio (RV/TLC). Hyperinflation is believed to be responsible, at least in part, for the sense of dyspnea.

**Exercise capacity:** Reduced capacity for exercise is a typical consequence of airflow obstruction in COPD patients, particularly because of dynamic hyperinflation occurring during exercise. Assessment of exercise capacity by treadmill or cycle ergometry combined with lung volume assessment is in some cases a tool to assess efficacy of a COPD drug. Alternative assessments of exercise capacity, such as the Six Minute Walk or Shuttle Walk, can also be used in some cases. The characteristics, including the limitations, of these tests will be known to those skilled in the art.

Outcome Measures can also be used, alone or preferably in combination with one or more objective tests, to determine efficacy of COPD therapy.

**Symptom Scores:** Symptom scores determined by asking patients to evaluate specific symptoms on a categorical, visual or numerical scale can be a simple way to assess efficacy of a drug based on the patient's own assessment of health status. Symptom scores can be valuable for assessing efficacy of a drug specifically aimed at relieving a symptom. In clinical programs aimed at other aspects of COPD, patient-reported symptom scores can be useful in assessing secondary effects of the therapy and may provide important additional evidence of efficacy. The characteristics, including the limitations, of these tests will be known to those skilled in the art.

**Activity Scales:** Activity scales such as the Medical Research Council dyspnea score, the Borg Scale, and the Mahler Baseline Dyspnea Index/Transitional Dyspnea Index, can be used in some cases as supportive evidence of efficacy. These scales are relatively simple to administer. The characteristics, including the limitations, of these tests will be known to those skilled in the art.

**Health-related, quality-of-life instruments:** Health-related quality-of-life instruments, such as the St. George's Respiratory Questionnaire and the Chronic Respiratory Questionnaire, are designed to systematically assess many different aspects of the effect of COPD on a patient's life. These instruments can be used to assess efficacy of a drug. These instruments are multidimensional and assess various effects of the disease on a patient's life and health status. The characteristics, including the limitations, of these tests will be known to those skilled in the art.

Further information regarding testing drugs for efficacy in the treatment of COPD can be found in the United States Food and Drug Administration's guidance document entitled: "Guidance for Industry: Chronic Obstructive Pulmonary Disease: Developing Drugs for Treatment," November, 2007, which is available from www.fda.gov/cder/guidance/index.htm.

A muscarinic antagonist is said to have a therapeutic effect in the treatment of COPD when it causes an increase in one or more measures of pulmonary function to a predetermined percentage above baseline. In some embodiments, the predetermined percentage above baseline is about 5%, about 10%, about 15%, about 20%, or about 25%. In some specific embodiments, a muscarinic antagonist will be considered to have a therapeutic effect when it raises one or more of the above-mentioned spirometry measurements (e.g. FEV₁) at least about 15% above baseline.

Spirometry is the measurement of respiration, which is generally conducted by a physician with the aid of a spirometer. Spirometers measure inspired and expired airflow for the purpose of assessing pulmonary ventilatory function. Spirometry is the most common pulmonary function test measuring lung function. Typical spirometers display volume-time curves (showing volume on the Y-axis and time, usually in seconds, on the X-axis) and optionally a flow-volume curves (showing rate of flow on the Y-axis and the total volume inspired/expired on the X-axis). U.S. Pat. No. 7,291,115 discloses a spirometer and method to measure the ventilatory function by spirometry, and is hereby incorporated by reference in its entirety. Methods of using a spirometer are familiar to those skill in the art.

Relevant parameters measured by spirometers include:
- FEV1 (or FEV₁): Forced Expiratory Volume in 1 Second, which is the maximum volume of air exhaled during the first second of maximum effort from a maximum inhalation. It is expressed in liters and in percentage of the patient's reference value from baseline. It becomes altered in cases of bronchial obstruction and it is fundamental for diagnosing and monitoring obstructive diseases, e.g. COPD.
- Change in FEV₁: Change in FEV₁ may be calculated as the difference between the FEV₁ value measured after dosing and the FEV₁ measured immediately prior to dosing. Change in FEV₁ may also be measured in reference to a placebo. These values may be expressed in absolute terms or in terms of percent change from baseline or placebo.
- FEV₁ AUC: This is the area between the FEV1 measurements vs. time curve over a time course. In some embodiments, the time course is a predetermined period, such as 12 hr., 18 hr., 24 hr., 30 hr., or 36 hr.
- Trough FEV₁: This is the FEV₁ value measured just prior to administration of the drug. In some cases, the trough FEV₁ is obtained in the morning, just prior to administration of the drug. In some embodiments, the change in trough FEV₁ is the difference between the trough FEV₁ for the drug and the trough FEV₁ for a placebo, after a period of time. In some embodiments, the change in the trough FEV₁ is measured over a predetermined time course, such as 1 wk, 2 wk, 4 wk or 12 wk.
- FVC: Forced Vital Capacity, which is the maximal volume of air exhaled with maximal effort from a position of maximal inhalation. It is expressed in liters and in percentage of a patient's reference value from baseline.
- FEV₁ /FVC: The quotient of FEV₁ and FVC.
- PEF: Peak Expiratory Flow, which is the highest expiratory flow achieved with maximal effort from a position of maximal inspiration. This is essentially the speed of the air moving out of the lungs of a patient at the beginning of expiration. It is expressed in liters/second or in liters/minute.
- FEF₂₅₋₇₅: Forced Expiratory Flow from 25% to 75% on the flow-volume curve, which is the average flow (or speed) of air coming out of the lung during the middle portion of expiration.
- FEF₂₅₋₅₀: Forced Expiratory Flow from 25% to 50% on the flow-volume curve, which is another measure of the average flow (or speed) of air coming out of the lung during the initial portion of expiration.
- FIF₂₅₋₇₅: Forced Inspiratory Flow from 25% to 75% on the flow-volume curve, which is the average flow (or speed) of air entering the lung during the middle portion of inspiration.
- FIF₂₅₋₅₀: Forced Inspiratory Flow from 25% to 50% on the flow-volume curve, which is another measure of the average flow (or speed) of air entering the lung during the initial portion of inspiration.

An enhanced therapeutic effect can include an increased magnitude of therapeutic effect, an enhanced duration of therapeutic effect, an enhanced time to onset of therapeutic effect, a shorter time to maximum therapeutic effect or a greater magnitude of therapeutic effect. In some embodiments described herein, an enhanced therapeutic effect relates to the increased ability of a pharmaceutical agent to relieve the symptoms of an airway respiratory disorder, e.g. COPD. Thus, an enhanced therapeutic effect may be determined by comparing values of change in FEV₁ (i.e. change in FEV₁ from baseline or compared to a placebo), % change in FEV₁ (i.e. percent change in FEV₁ from baseline or compared to placebo), FEV₁ AUC, trough FEV₁, FEV₁/FVC, PEF, FEF₂₅₋₇₅, FEF₂₅₋₅₀, FIF₂₅₋₇₅, FIF₂₅₋₅₀ obtained from a patient or patient population in one therapeutic milieu versus another anther therapeutic milieu. For example, an enhanced therapeutic effect may be determined by comparing FEV₁ values for a patient or patient population treated with a muscarinic antagonist administered with a high efficiency nebulizer against the same drug administered with a conventional nebulizer. In another example, an enhanced therapeutic effect may be determined by comparing FEV₁ values for a patient or patient population treated with a muscarinic antagonist administered at a high concentration against the same drug administered at a low concentration. In some cases, an enhanced therapeutic effect may be determined by comparing FEV₁ values for a patient or patient population treated with a muscarinic antagonist administered with a high efficiency nebulizer against a muscarinic antagonist alone administered with a conventional nebulizer. In another example, an enhanced therapeutic effect may be determined by comparing FEV₁ values for a patient or patient population treated with a muscarinic antagonist administered at a high concentration against a muscarinic antagonist alone administered at a low concentration. In some embodiments, the enhanced therapeutic effect is an increased magnitude of therapeutic effect. In some embodiments, the increased magnitude of therapeutic effect is an increase in the peak FEV₁ obtained with a high efficiency nebulizer versus the peak FEV₁ obtained with a conventional nebulizer. In some embodiments, the peak FEV₁ obtained with a high efficiency nebulizer is at least about 5%, 10%, or 15% above that obtained with a conventional nebulizer. In some embodiments, the peak FEV₁ obtained with a high efficiency nebulizer is at least about 50 mL, 100 mL, or 150 mL above that obtained with a conventional nebulizer. In some embodiments, the increased magnitude of therapeutic effect is an increase in the mean FEV₁ obtained with a high efficiency nebulizer versus the mean FEV₁ obtained with a conventional nebulizer. In some embodiments, the mean FEV₁ obtained with a high efficiency nebulizer is at least about 5%, 10%, or 15% above that obtained with a conventional nebulizer. In some embodiments, the mean FEV₁ obtained with a high efficiency nebulizer is at least about 50 mL, 100 mL, or 150 mL above that obtained with a conventional nebulizer. In some embodiments, the increased magnitude of therapeutic effect is an increase in the AUC for the FEV₁ versus time curve obtained with a high efficiency nebulizer versus the AUC for the FEV₁ versus time curve obtained with a conventional nebulizer. In some embodiments, the increase in AUC of the FEV₁ versus time curve obtained with a high efficiency nebulizer is at least about 5%, 10%, or 15% above that obtained with a conventional nebulizer.

In some embodiments, the method or system (e.g. muscarinic antagonist, optionally in combination with a beta 2-adrenergic agonist, administered at a high concentration and/or with a high efficiency nebulizer) provides an enhanced duration of therapeutic effect, as determined by the amount of time that a spirometric parameter (e.g. FEV₁) is above a predetermined threshold after therapy is administered. In some embodiments, the predetermined threshold is at least about 5% above baseline, at least about 10% above baseline, at least about 15% above baseline, at least about 20% above baseline, at least about 25% above baseline. In some specific embodiments, the threshold is about 15% above baseline. In some specific embodiments, the threshold is about 10% above baseline. In some embodiments, the threshold is 50 mL, 100 mL, 150 mL or more than about 150 mL above baseline. In some specific embodiments, the threshold is about 100 mL above baseline. Baseline can be determined by either a one-time reference to the spirometric parameter (e.g. FEV₁) immediately prior to administration of API, or by reference to the spirometric parameter level at several time periods during the study following administration of placebo to a predetermined set of patients. In some embodiments, baseline is determined based on the level of spirometric parameter (e.g. FEV₁) immediately prior to administration to the patient of muscarinic antagonist administered at a high concentration and/or with a high efficiency nebulizer. In some embodiments, baseline is determined by reference to the level of spirometric parameter (e.g. FEV₁) at several time periods during evaluation of certain patients following placebo administration, with the simultaneous evaluation of other patients administered a muscarinic antagonist administered at a high concentration and/or with a high efficiency nebulizer.

In some embodiments, a duration of therapeutic effect is the period during which FEV₁ is at least about 5% above baseline, at least about 10% above baseline, at least about 15% above baseline, at least about 20% above baseline, at least about 25% above baseline. In some specific embodiments, the duration of therapeutic effect is the amount of time that the FEV₁ is at least 15% above baseline. In some specific embodiments, the duration of therapeutic effect is the amount of time that the FEV₁ is at least 10% above baseline. In some specific embodiments, the duration of therapeutic effect is the amount of time that the FEV₁ is at least 50 mL, 100 mL, or 150 mL above baseline. In some embodiments, a duration of therapeutic effect is the period during which FEV₁/FVC is at least about 5% above baseline, at least about 10% above baseline, at least about 15% above baseline, at least about 20% above baseline, at least about 25% above baseline. In some embodiments, the duration of therapeutic effect is the amount of time that the FEV₁/FVC is at least 15% above baseline. In some embodiments, a duration of therapeutic effect is the period during which PEF is at least about 5% above baseline, at least about 10% above baseline, at least about 15% above baseline, at least about 20% above baseline, at least about 25% above baseline. In some embodiments, the duration of therapeutic effect is the amount of time that the PEF is at least 15% above baseline. In some embodiments, a duration of therapeutic effect is the period during which FEF₂₅₋₇₅ is at least about 5% above baseline, at least about 10% above baseline, at least about 15% above baseline, at least about 20% above baseline, at least about 25% above baseline. In some embodiments, the duration of therapeutic effect is the amount of time that the FEF₂₅₋₇₅ is at least 15% above baseline. In some embodiments, a duration of therapeutic effect is the period during which FEF₂₅₋₅₀ is at least about 5% above baseline, at least about 10% above baseline, at least about 15% above baseline, at least about 20% above baseline, at least about 25% above baseline. In some embodiments, the duration of therapeutic effect is the amount of time that the FEF₂₅₋₅₀ is at least 15% above baseline. In some embodiments, a duration of therapeutic effect is the period during which FIF₂₅₋₇₅ is at least about 5% above baseline, at least about 10% above baseline, at least about 15% above baseline, at least about 20% above baseline, at least about 25% above baseline. In some embodiments, the duration of therapeutic effect is the amount of time that the FIF₂₅₋₇₅ is at least 15% above baseline. In some embodiments, a duration of therapeutic effect is the period during which FIF₂₅₋₅₀ is at least about 5% above baseline, at least about 10% above baseline, at least about 15% above baseline, at least about 20% above baseline, at least about 25% above baseline. In some embodiments, the duration of therapeutic effect is the amount of time that the FIF₂₅₋₅₀ is at least 15% above baseline.

A significantly greater, or greater, duration of therapeutic effect, indicates that the method or system (e.g. a high efficiency nebulizer-administered muscarinic antagonist) provides an increased period of time the spirometric parameter is above a predetermined threshold of about 5% above baseline, about 10% above baseline, about 15% above baseline, about 20% above baseline, about 25% above baseline, especially about 15% above baseline, for one or more of the spirometric parameters compared to the same spirometric parameter obtained with substantially the same nominal dose of drug administered with a different inhalation device, e.g. a conventional nebulizer.

"About the same" duration of therapeutic effect means that the method or system (e.g. a high efficiency nebulizer-administered muscarinic antagonist, optionally in combination with a beta 2-adrenergic agonist) provides substantially the same period of time that the spirometric parameter is above a predetermined threshold of about 5% above baseline, about 10% above baseline, about 15% above baseline, about 20% above baseline, about 25% above baseline, or especially about 15% above baseline, for one or more of the above spirometric parameters compared to the same spirometric parameter obtained with a substantially greater nominal dose of the muscarinic antagonist administered with a different inhalation device, e.g. conventional nebulizer (reference administration).

In some embodiments, an inhalation solution described herein (e.g. a muscarinic antagonist inhalation solution administered with a high efficiency nebulizer and/or at a high concentration) provides a duration of therapeutic effect of at least about 12 hr, about 12 hr to about 24 hr, about 18 hr to about 24 hr, about 20 hr to about 24 hr, or at least about 24 hr, in some embodiments.

A time to onset of therapeutic effect is the time for the spirometric parameter to reach a predetermined threshold of about 5% above baseline, about 10% above baseline, about 15% above baseline, about 20% above baseline, or about 25% above baseline, especially about 15% above baseline for one or more of the spirometric parameters of a muscarinic antagonist administered with an inhalation device. An enhanced time to onset of therapeutic effect relates to the increased ability of a pharmaceutical agent to relieve the symptoms of an airway respiratory disorder, e.g. COPD. The enhanced time to onset of therapeutic effect may be a measure of the FEV₁, FEV₁ /FVC, PEF, FEF₂₅₋₇₅, FEF₂₅₋₅₀, FIF₂₅₋₇₅, FIF₂₅₋₅₀ levels.

A significantly shorter, or shorter, time to onset of therapeutic effect, in some embodiments, means that the method or system (a muscarinic antagonist inhalation solution administered with a high efficiency nebulizer and/or at a high concentration) provides for a shortened period of time for one or more spirometric parameters (e.g. FEV₁) to reach a predetermined threshold of about 5% above baseline, about 10% above baseline, about 15% above baseline, about 20% above baseline, or about 25% above baseline, especially about 15% above baseline, for one or more of the spirometric parameters compared to the same spirometric parameter(s) obtained with substantially the same nominal dose of the drug solution administered with a different inhalation device, e.g. a conventional nebulizer and/or at a lower concentration. In other embodiments, "about the same" time to onset of therapeutic effect means the method or system (e.g. administration of a muscarinic antagonist with a high efficiency nebulizer and/or at a high concentration) provides for substantially the same period of time for the spirometric parameter to reach a predetermined threshold of about 5% above baseline, about 10% above baseline, about 15% above baseline, or about 20% above baseline for one or more of the spirometric parameters compared to the same spirometric parameter obtained with a substantially greater nominal of the dose the drug solution administered with a different inhalation device, e.g. a conventional nebulizer.

An inhalation solution that provides an onset of therapeutic effect of less than about 30 minutes, less than about 25 minutes, less than about 20 minutes, less than about 15 minutes, or less than about 10 minutes, in some embodiments, refers to an amount of time for the spirometric parameter to reach a predetermined threshold of about 5% above baseline, about 10% above baseline, about 15% above baseline, or about 20% above baseline.

In some embodiments, the methods or systems are provided for the treatment of acute exacerbations of Chronic Obstructive Pulmonary Disease (COPD), chronic bronchitis, and/or emphysema in a patient, comprising administering to the patient a nominal dose of a muscarinic antagonist in an aqueous inhalation solution at a concentration of a muscarinic antagonist sufficient to provide a rapid onset of therapeutic effect and a long duration of therapeutic effect. In some embodiments, the rapid onset of therapeutic effect is less than about 30 minutes, less than about 25 minutes, less than about 20 minutes, less than about 15 minutes or less than about 10 minutes. In some embodiments, the long duration of therapeutic effect is at least about 12 hr to about 24 hr, about 18 hr to about 24 hr, about 20 hr to about 24 hr or at least about 24 hr.

A time to maximum therapeutic effect means the amount of time for a preselected spirometric parameter to reach its peak level. In some embodiments, an enhanced time to maximum therapeutic effect means that administration of a muscarinic antagonist with a high efficiency nebulizer, at a high concentration or both, results in a faster time to maximum therapeutic effect than would a dose of the muscarinic antagonist administered with a conventional nebulizer or at a lower concentration. The parameters used to determine an enhanced time to maximum therapeutic effect may be one or more of FEV₁, FEV₁/FVC, PEF, FEF₂₅₋₇₅, FEF₂₅₋₅₀, FIF₂₅₋₇₅, or FIF₂₅₋₅₀.

### Reduction in Adverse Side Effects

Conventional COPD therapy employing a muscarinic antagonist with a conventional nebulizer often results in deposition of pharmaceutically active ingredient in sections distinct from the lung, e.g., mouth, throat, stomach, and/or esophagus. This results in antagonism of muscarinic receptors on peripheral systems other than the lung, for example in salivary glands, stomach, and elsewhere. Therefore the tolerated doses of systemically active muscarinic antagonists, such as glycopyrrolate, are limited by side-effects such as, but not limited to, xerostomia (dry mouth), urinary hesitancy and retention, blurred vision, tachycardia, dizziness, insomnia, impotence, mental confusion, excitement, headache, anxiety, hypotension and/or palpitations.

In the present invention, the bronchodilation and other beneficial actions of a muscarinic antagonist are produced by an inhaled agent providing for a high therapeutic index for activity in the lung, i.e. lung deposition, compared with deposition of muscarinic antagonist in non-pulmonary regions, i.e. periphery compartments, mouth and pharynx. The present invention further provides for an inhalable muscarinic antagonist with low bioavailability in areas within a patient other than the lung (e.g. systemic bioavailability, local oropharyngeal or gastric regions), resulting in a decreased incidence and/or severity of systemic and/or local toxicity and/or side effects. A practitioner of ordinary skill can quantify a reduction in adverse side effects by measuring the incidence and/or severity of systemic and/or local toxicity and/or side effects in a given patient or patient population.

A reduced, or decreased, incidence or severity of systemic and/or local toxicity and/or side effects means that the method or system (e.g. muscarinic antagonist, optionally in combination with a beta 2-adrenoreceptor agonist, administered with a high efficiency nebulizer and/or at a high concentration) provides a decreased incidence and/or severity of systemic and/or local toxicity and/or side effects (for example dry mouth) in a given patient or patient population compared to a given reference therapy. In some embodiments, the reference therapy is administration of a muscarinic antagonist with a conventional nebulizer. Some embodiments provide a method for the treatment of COPD in a patient, comprising administering to the patient a nominal dose of muscarinic antagonist which, when administered with a high efficiency nebulizer, provides a calculated respirable dose of a muscarinic antagonist with a high efficiency nebulizer, wherein the calculated respirable dose of the muscarinic antagonist administered with the high efficiency nebulizer demonstrates a decreased incidence and/or severity of systemic and/or local toxicity and/or side effects in the patient as compared to a nominal dose that achieves substantially the same calculated respirable dose of the muscarinic antagonist administered with a conventional nebulizer. Some embodiments provide a method for the treatment of COPD in a patient, comprising administering to the patient a nominal dose of muscarinic antagonist which, when administered with a high efficiency nebulizer, provides a deposited lung dose of a muscarinic antagonist with a high efficiency nebulizer, wherein the deposited lung dose of the muscarinic antagonist administered with the high efficiency nebulizer demonstrates a decreased incidence and/or severity of systemic and/or local toxicity and/or side effects in the patient as compared to a nominal dose that achieves substantially the same deposited lung dose of the muscarinic antagonist administered with a conventional nebulizer. Some embodiments provide a system for performing the foregoing methods.

In some embodiments, the method or system (e.g. administration of a muscarinic antagonist with a high efficiency nebulizer and/or at a high concentration) provides a method and/or inhalation system for administration of a muscarinic antagonist in a volume of about 0.5 mL or less, 1 mL or less, 1.5 mL or less, or 2.0 mL or less and wherein the muscarinic antagonist demonstrates less incidence and/or severity of systemic and/or local toxicity and/or side effects (for example dry mouth) in the patient as compared to substantially the same nominal dose of the muscarinic antagonist administered in a substantially higher volume of solution.

In some embodiments, the method or system (e.g. muscarinic antagonist with a high efficiency nebulizer and/or at a high concentration) provides for methods and inhalation systems for reducing at least one side effect of the muscarinic antagonist and providing a duration of therapeutic effect of at least about 12 hr, about 12 hr to about 24 hr, about 18 hr to about 24 hr, about 20 hr to about 24 hr, or at least about 24 hours. In some embodiments, the method or system (e.g. administration of a muscarinic antagonist with a high efficiency nebulizer and/or at a high concentration) provides for co-administration of other drugs and optionally excipients, for example an organic acid, such as ascorbic acid, citric acid or a mixture of both, pilocarpine, cevimeline or carboxymethylcellulose, or a mucolytic compound.

### Enhanced Lung Deposition

Muscarinic receptors and beta 2-adrenoreceptors are widely distributed throughout the body. The ability to apply these active pharmaceutical agents (APIs) locally to the respiratory tract with sufficient lung deposition is particularly advantageous, as it would allow for administration of lower doses of the drug fostering increased patient compliance

The principle advantage of administration of a nebulized API solution with a high efficiency nebulizer over other methods of pulmonary delivery of APIs is that the methods and systems described herein offer more efficient delivery of higher doses of API compared to conventional inhalation methods and systems, resulting in greater efficacy and a reduced incidence and optionally a severity of side effects in the patient. A more efficient delivery of API is evidenced by direct delivery and deposition of an API to the site of action, i.e. the lung (as used herein, "lung" refers to either or both the right and left lung organs). It can be assumed that substantially all of an API delivered at the receptor site in the lungs will be absorbed into the blood plasma of the patient. In embodiments of the invention, the API is a muscarinic antagonist (optionally in combination with a beta 2-adrenoreceptor agonist), such as glycopyrronium bromide (glycopyrrolate).

The deposited dose may be expressed in terms of lung deposition. A lung deposition of 30% means 30% of the active ingredient in the inhalation device just prior to administration is deposited in the lung. Likewise, a lung deposition of 60% means 60% of the active ingredient in the inhalation device just prior to administration is deposited in the lung, and so forth. Lung deposition (deposited lung dose) can be determined using methods of scintigraphy or deconvolution. In some embodiments, the present invention provides for methods and inhalation systems for the treatment or prophylaxis of a respiratory condition in a patient, comprising administering to the patient a nominal dose of a muscarinic antagonist solution with a high efficiency nebulizer wherein administration of the muscarinic antagonist with the inhalation device provides lung deposition (deposited lung dose) of the muscarinic antagonist of at least about 30%, at least about 35%, at least about 40%, at least about 45%, at least about 50%, at least about 55%, at least about 60%, about 30% to about 60%, about 30% to about 55%, about 30% to about 50%, about 30% to about 40%, about 30% to about 90%, about 40% to about 80%, about 50% to about 60%, or about 60% to about 70% based on the nominal dose of the muscarinic antagonist. In some embodiments, the present invention provides for methods and inhalation systems for the treatment or prophylaxis of a respiratory condition in a patient, comprising administering to the patient a nominal dose of a muscarinic antagonist in an aqueous inhalation solution with an inhalation device wherein administration of the muscarinic antagonist with the inhalation device provides lung deposition (deposited lung dose) of the muscarinic antagonist of at least about 15%, at least about 20%, at least about 25%, at least about 30%, at least about 35%, at least about 40%, at least about 45%, at least about 50%, at least about 55%, at least about 60%, about 20% to about 40%, about 25% to about 35%, about 25% to about 30%, about 35% to about 90%, about 40% to about 80%, about 50% to about 60%, or about 60% to about 70% based on the nominal dose of the muscarinic antagonist.

Aerosol particle/droplet size is one of the most important factors determining the deposition of aerosol drugs in the airways. The portion of an aerosol that has the highest probability of bypassing the upper airway and depositing in the lung measure between 1 and 5 µm. Particles larger than this are generally deposited in the oropharyngeal region and are swallowed, while sub-micron particles do not carry much drug and may be exhaled before deposition takes place. Smaller particles tend to deposit more peripherally in the lung than coarser particles, which may lead to a different clinical response. Consequently, differences in particle size of the aerosol emitted from inhalation devices may account for some of the variability in therapeutic efficacy and safety. Measurement of particle size, therefore, has an important role in guiding product development and in quality control of the marketed product.

The distribution of aerosol particle/droplet size can be expressed in terms of either or both of:
- The Mass Median Aerodynamic Diameter (MMAD) and the Geometric Standard Deviation (GSD), wherein the MMAD is the droplet size at which half of the mass of the aerosol is contained in smaller droplets and half in larger droplets and the GSD is the geometric standard deviation of the particle population
- The Fine Particle Fraction (FPF), which is the fraction of particles (which may be expressed as a percentage) that are <5 µm in diameter.

These measures have been used for comparisons of the in vitro performance of different inhaler device and drug combinations. In general, the higher the fine particle fraction, the higher the proportion of the emitted dose that is likely to reach the lung.

There are two main methods used to measure aerosol deposition in the lungs. First, γ-scintigraphy is performed by radiolabeling the drug with a substance like 99m-technetium, and scanning the subject after inhalation of the drug. This technique has the advantage of being able to quantify the proportion of aerosol inhaled by the patient, as well as regional distribution in the upper airway and lungs. Second, since most of the drug deposited in the lower airways will be absorbed into the bloodstream, pharmacokinetic techniques are used to measure lung deposition (deposited lung dose). This technique can assess the total amount of drug that interacts with the airway epithelium and is absorbed systemically, but will miss the small portion that may be expectorated or swallowed after mucociliary clearance, and cannot tell us about regional distribution. Therefore, γ-scintigraphy and pharmacokinetic studies are in many cases considered complementary.

The relationship between pulmonary deposition of inhaled β2-agonists and therapeutic effect is now well-established, since the immediate effects of these agents on the airways are relatively easy to measure. As the pulmonary dose-response curve for the β2-agonists is sigmoidal (i.e. an initial slope followed by a plateau), increasing the dose deposited in the lung will elicit an increased therapeutic effect only if the initial dose was on the rising slope of the dose-response curve.

Lung deposition of a particular drug is influenced by the mass of drug contained in the nebulized droplets administered to a patient with a particular Mass Median Aerodynamic Diameter (MMAD) and Geometric Standard Deviation (GSD). In general, there is an inverse relationship between the average MMAD and GSD of a particular nebulizer's emitted droplets and deposition of the droplets in a patient's lung. A smaller MMAD results in an increased likelihood of lung deposition in a patient. When the MMAD is in the range of about 3.0-4.5 µm, a narrower GSD results in a higher degree of lung deposition, since a higher percentage of particles will be under 5 µm in diameter. It is believed that, in general, aerosol particles greater than -10 µm in aerodynamic diameter deposit primarily in the oropharynx and are swallowed rather than reaching the lungs. Because of the plausible link between MMAD and GSD values and eventual deposition site within the respiratory tract, smaller MMAD and GSD values may affect both the safety (by reducing non-pulmonary deposition and possibly thereby reducing local and potentially systemic effects) and the efficacy (by increasing the amount of drug actually deposited in the lungs) of drug products administered with such high efficiency inhalation devices. Laser-diffraction provides for an *in vitro* method of determining MMAD and GSD data, which can then be plotted onto what usually results in a log-normal shaped curve (depicting mass distribution % on the Y-axis and droplet diameter on the X-axis). Laser-diffraction methods are well-known to one of ordinary skill in the art. In addition to laser-diffraction methods, *in vitro* data for MMAD and GSD can also be measured using cascade impaction or time-of-flight analytical methods, both of which are known to one of ordinary skill in the art.

Geometric Standard Deviation (GSD) is a dimensionless measure of dispersion from a geometric mean, such as the MMAD. In general, the smaller the GSD for a particular particle size distribution, the narrower the distribution curve. In some embodiments, administration of the muscarinic antagonist with the high efficiency nebulizer provides a GSD of emitted droplet size distribution of the solution administered with a high efficiency nebulizer of about 1.1 to about 2.1, about 1.2 to about 2.0, about 1.3 to about 1.9, less than about 2, at least about 1.4 to about 1.8, at least about 1.5 to about 1.7, about 1.4, about 1.5, or about 1.6. In some embodiments, administration of API with a high efficiency nebulizer provides a Mass Median Aerodynamic Diameter (MMAD) of droplet size of the solution emitted with the high efficiency nebulizer of about 1 µm to about 5 µm, about 2 to about 4 µm, about 3 to about 4 µm, or about 3.5 to about 4.5 µm.

Respirable Fraction (RF), Emitted Dose (ED), Respirable Dose (RD) and the Respirable Dose Delivery Rate (RDDR) are *in* vitro-derived (calculated) parameters that provide technical dimensions for the efficiency of a nebulizer inhalation device. RF, which is measured with a cascade impactor or laser diffraction apparatus, is a generally accepted estimate within the medical community of the fraction, which may be expressed as a percentage, of drug that is available for lung deposition. RF represents the percentage of the delivered aerosol dose, or inhaled mass, with droplets of diameter less than 5.0 µm. Droplets of less than 5.0 µm in diameter are considered to penetrate to the lung. In some embodiments, administration of the muscarinic antagonist with a high efficiency nebulizer provides a respirable fraction (RF) of API of at least about 60%, at least about 65%, at least about 70%, at least about 75%, at least about 80%, at least about 85%, at least about 90%, about 60% to about 95%, about 65% to about 95%, or about 70% to about 90%. In some embodiments, the high efficiency nebulizer is characterized by an RF of at least about 80% or about 70% to about 90%.

The Emitted Dose (ED) of drug administered to a patient is the portion of volume of liquid filled into the nebulizer, i.e. the fill volume, which is actually emitted from the mouthpiece of the device. The difference between the nominal dose and the ED is the amount of volume lost primarily to residues, i.e. the amount of fill volume remaining in the nebulizer after administration, or is lost in aerosol form. The ED of the muscarinic antagonist is to be tested under simulated breathing conditions using a standardized bench setup, which are known to one of skill in the art. In some embodiments, the ED of the muscarinic antagonist of the present invention is at least about 30%, at least about 35%, at least about 40%, at least about 45%, at least about 50%, at least about 55%, at least about 60%, about 30% to about 60%, about 30% to about 55%, about 30% to about 50%, about 30% to about 40%, about 30% to about 75%, about 40% to about 70%, or about 45% to about 60%. The Respirable Dose (RD) is an expression of the delivered mass of drug contained within emitted droplets from a nebulizer that are small enough to penetrate the lung of a patient. The RD is determined by multiplying the ED by the RF, and can be readily determined using the respective ED and RF values provided herein.

The output rate is the speed at which API is administered from the inhalation device. In some embodiments, administration of the muscarinic antagonist with the high efficiency nebulizer provides an output rate of API of at least about 2 times, 3 times or 4 times the output rate achievable with a conventional nebulizer. For example, where the muscarinic antagonist is glycopyrrolate, in some embodiments the output rate is at least about 120 µg/min, at least about 150 µg/min, at least about 200 µg/min or at least about 200 µg/min to at least about 5,000 µg/min. In some embodiments, administration of glycopyrrolate with the high efficiency nebulizer provides an output rate of API of at least about 120 µL/min, at least about 150 µL/min, at least about 200 µL/min or at least about 200 µL/min to at least about 5,000 µL/min.

The Respirable Dose Delivery Rate (RDDR) is the speed at which a respirable dose of the drug is nebulized, administered, and delivered to a patient's lungs. RDDR, measured as a function of µg/min, is determined by dividing the RD (in µg) by the amount of time necessary for inhalation. The amount of time necessary for inhalation is measured as the amount of time from the first moment of administration of the emitted droplet from the nebulizer until the emitted droplet of respirable diameter is delivered to the lung, as measured using a standardized bench setup simulating breathing conditions. In some embodiments, administration of the muscarinic antagonist to the patient with the high efficiency nebulizer provides a respirable dose delivery rate (RDDR) of at least about 2 times, 3 times or 4 times a delivery rate achievable with a conventional nebulizer. Where the muscarinic antagonist is glycopyrrolate, in some embodiments the RDDR is at least about 100 µg/min, at least about 150 µg/min, at least about 200 µg/min, about 100 µg/min to about 5,000 µg/min, about 150 µg/min to about 4,000 µg/min or about 200 µg/min to about 3,500 µg/min. In some embodiments, administration of the glycopyrrolate to the patient with an aqueous inhalation device provides an RDDR of at least about 100 µg/min or about 100 µg/min to about 5,000 µg/min.

### EXAMPLES

The following ingredients, processes and procedures for practicing the systems and methods disclosed herein correspond to that described above. The procedures below describe some embodiments of methods of delivery of a nebulized long-acting cholinergic antagonist aqueous solution (as a monotherapeutic agent), or in combination with a nebulized B2-adrenoreceptor aqueous solution (in combination therapy) as described herein and pharmacokinetic profiles thereof. Methods, materials, or excipients which are not specifically described in the following examples are within the scope of the invention and will be apparent to those skilled in the art with reference to the disclosure herein.

### Example 1: Single-Dose, Dose Escalation Study to Assess Glycopyrrolate Inhalation Solution (GIS) Using a high efficiency nebulizer in Patients with COPD

### Objectives:

The objectives of the study are as follows: *Primary*: To assess the safety and tolerability of single ascending doses of glycopyrrolate inhalation solution when administered using a high efficiency nebulizer in patients with COPD. *Secondary*: (1) To assess and to compare the magnitude and duration of bronchodilator response in patients with COPD following single doses of glycopyrrolate inhalation solution when administered using a high efficiency nebulizer and a general purpose nebulizer; and (2) To assess the pharmacokinetic (PK) profile of glycopyrrolate.

### Methodology:

This two part study is a single-center, single dose, dose-escalation study in patients with COPD of 45-65 years of age. At least 12 subjects are included in the study.

Part 1 is an open label study to assess the safety, tolerability, PK profile and bronchodilator response following single doses of glycopyrrolate inhalation solution when administered via a high efficiency nebulizer. Subjects are studied in two cohorts in parallel; two doses of glycopyrrolate inhalation solution are administered in each cohort in a dose-escalation manner at two separate visits with a washout period of 5 to 10 days. If 24 hours of bronchodilation is not achieved at the doses tested, an additional higher dose of glycopyrrolate inhalation solution is administered on a separate visit. After establishing the minimum effective dose of glycopyrrolate inhalation solution that provides approximately 24 hours of bronchodilation when using a high efficiency nebulizer in Part 1, all subjects enter Part 2 of the study to receive in a randomized, double-blind manner a single dose of either the minimum effective dose of glycopyrrolate inhalation solution (as determined in Part 1) or placebo using a general purpose jet nebulizer. The high efficiency nebulizer tested is eFlow^{®} (PARI, Germany).

Within 14 days following a Screening Visit, eligible subjects are randomly allocated to one of two cohorts of 4 subjects each at the Baseline Visit and undergo baseline assessments, lung function assessments. During the Treatment Period, subjects receive study treatments as a single dose treatment with a washout period of 5-10 days between each Treatment Visit.

Study procedures during each Treatment Period include serial spirometry (including FEV₁, FVC and PEFR) immediately following dosing (within 5 minutes) and 15, 30 60, 90 minutes and 2, 4, 6, 8, 10, 12, 15, 18, 21, 24, 27, 30, 33 and 36 hours post-dose. Blood samples are obtained to determine plasma GP concentrations at pre-dose and 5, 15, 30, 45 minutes and 1, 2, 4, 6, 8 and 12 hours post-dose. Vital sign and 12-lead electrocardiogram are obtained at pre dose and 30 and 60 minutes and 2, 4, 8, 12 and 24 hours post-dose. Clinical labs are done at the Screening Visit and within 7 days following the last study treatment (Safety Follow-up Visit).

Subjects are required to withhold any long-acting beta2-agonist bronchodilators (e.g., formoterol or salmeterol) for 24 hours and short-acting beta2 agonist bronchodilators (e.g. salbutamol) for 8 hours prior to receiving each study treatment. Inhaled anti-cholinergic bronchodilators (e.g., tiotropium) are not permitted within 5 days prior to Screening Visit and for the duration of the study. Inhaled steroid use are permitted during the study provided that the steroid dose is maintained unchanged. Rescue salbutamol is allowed for as-needed use any time during the study. However, if rescue salbutamol is used within 8 hours of the Treatment Visit, then the visit is rescheduled.

### Main Criteria for Inclusion and Exclusion

Subjects are eligible for the study if they meet the following inclusion criteria: 1. Age 45 through 65 years, inclusive; 2. A clinical diagnosis of COPD; 3. At least 10 pack-year smoking history (e.g., at least 1 pack/day for 10 years, or 10 packs/day for 1 year); 4. Pre-bronchodilator FEV₁ 40-80% of predicted normal; 5. Pre-bronchodilator FEV₁ /FVC ratio < 0.70; 6. Improvement in FEV₁ >12% (and 150 mL) following inhalation of ipratropium bromide; 7. Ability to perform reproducible spirometry according to the American Thoracic Society standards; 8. If female and of childbearing potential, must have a negative pregnancy test and not lactating at the Screening Visit, and must be using one of the following acceptable means of birth control throughout the study: (a) Abstinence; (b) Post-menopausal for at least two years; (c) Surgically sterile; (d) Oral contraceptives (taken for at least one month prior to the Screening Visit); (e) Approved implantable or injectable contraceptives (e.g., Norplant®, Depo-Provera® or equivalent); (f) Barrier methods (e.g., condoms with spermicide); (g) Intrauterine device (i.e., IUD); (h) Vasectomy of male partner; (i) Non-heterosexual life style; 9. Each subject must be willing and able to provide written informed consent

Exclusion criteria: 1. Current evidence or recent history of any clinically significant disease (other than COPD) or abnormality in the opinion of the Investigator that would put the subjects at risk or which would compromise the quality of the study data; including but not limited to cardiovascular disease, myocardial infarction, hypertension, arrhythmia, diabetes, neurological or neuromuscular disease, liver disease, gastrointestinal disease or electrolyte abnormalities; 2. Recent history of COPD exacerbation within the previous 12 months or need for increased treatments for COPD within 4 weeks prior to the Screening Visit; 3. Regular use of long-term oxygen therapy; 4. Use of oral or inhaled steroids within 3 months prior to the Screening Visit; 5. History of tuberculosis, bronchiectasis or other non-specific pulmonary disease; 6. History of urinary retention or bladder neck obstruction type symptoms; 7. History of narrow-angle glaucoma; 8. Current or recent history (previous 12 months) of excessive use or abuse of alcohol; 9. Current evidence or history of abusing legal drugs or use of illegal drugs or substances; 10. History of hypersensitivity or intolerance to aerosol medications; 11. Participation in another investigational drug study within 30 days prior to the Screening Visit.

### Study Treatments, Dose and Mode of Administration:

Part 1 (n=12): Subject is randomly allocated to one of 2 cohorts running in parallel.

Cohort 1 (n=6): 1. GIS 25 mcg/0.5 mL oral inhalation via eFlow; 2. GIS 200 mcg/0.5 mL oral inhalation via eFlow.

Cohort 2 (n=6): 1. GIS 75 mcg/0.5 mL oral inhalation via eFlow; 2. GIS 500 mcg/0.5 mL oral inhalation via eFlow.

If 24 hours of bronchodilation is not achieved at the doses tested (25-500 mcg/0.5 mL), then an additional dose of 1000 mcg/0.5 mL is administered on a separate visit.

### Duration of Study:

The total duration of the study is approximately 7 weeks, consisting of a Screening Period within 14 days before the first Treatment Visit, maximum of four Treatment Visits of approximately 36 hours each, maximum of three washout periods of 5-10 days each, and a Safety Follow-Up Visit within 7 days after the last study treatment.

### Criteria for Evaluation:

The primary efficacy variable is serial spirometry throughout the 36 hour period after dosing. The spirometry assessments include forced expiratory volume in one second (FEV₁), forced vital capacity (FVC), peak expiratory flow rate (PEFR), FEV₁ % of predicted normal.

The PK parameters to be evaluated for plasma glycopyrrolate are maximum concentration (Cₘₐₓ), time to maximum concentration (Tₘₐₓ), terminal elimination half-life (T_{1/2}), area under the plasma concentration-time curve from time = 0 to time of last measurable drug concentration (AUC₀₋ₜ), and area under the plasma concentration-time curve from time = 0 to infinity (AUC_{0-inf}).

Safety parameters include adverse events (AEs) including assessment of dry mouth, and changes in vital signs, 12-lead ECG (including QTc interval), and clinical laboratory tests.

Part 2 (n=12): All subjects participated in Part 1 are included. Glycopyrrolate inhalation solution (dose selected from Part 1) in 2 mL or Placebo oral inhalation via general purpose nebulizer.

### Statistical Measurements:

Analysis of variance is used to calculate point estimates, and confidence intervals (CI) for treatment differences with respect to spirometry assessments (95% CI) and some PK parameters (90% CI) are calculated.

The incidence of AEs is compared between treatment groups. Summary tables and individual subject listings are provided for all safety measurements and the results are presented by treatment group. Descriptive statistics are used to summarise data where appropriate.

### Example 2: Scintigraphy Study

### Objective

The objective of the study is assess and characterize the pulmonary deposition profile of glycopyrrolate inhalation solution when administered via a high efficiency nebulizer and a jet nebulizer in healthy volunteers using gamma scintigraphic images.

### Methodology

Subjects undergo a ventilation scan by gamma camera scintigraphy followed by a 30-minute washout period before receiving study treatment. Post-dosing procedures include a distribution scan by gamma scintigraphy performed immediately after inhalation dosing. Gamma scintigraphy using glycopyrrolate inhalation solution radiolabelled with an appropriate chelate, i.e. Technetium-99m Diethylenetriamine-penta acetic acid (99mTc-DTPA).

At least four (4) treatments are administered to each subject: (1) glycopyrrolate inhalation solution 0.25 mg/0.5 mL (radiolabelled with 99mTc-DTPA) oral inhalation via eFlow; (2) glycopyrrolate inhalation solution 0.5 mg/0.5 mL (radiolabelled with 99mTc-DTPA) oral inhalation via eFlow; (3) glycopyrrolate inhalation solution 0.5 mg/2 mL (radiolabelled with 99mTc-DTPA) oral inhalation via LC Plus; and (4) glycopyrrolate inhalation solution 1 mg/2 mL (radiolabelled with 99mTc-DTPA) oral inhalation via LC Plus. Additional treatments are administered at additional concentrations of glycopyrrolate if appropriate.

### Conclusions

The experiment demonstrates the superior deposition and distribution of an inhalation solution containing glycopyrrolate in the lungs when administered by a high efficiency nebulizer (eFlow^{®}) as compared to administration of the same nominal (loaded) dose of glycopyrrolate administered with a conventional jet nebulizer. While not wishing to be bound by theory, the inventor believes that the improved deposition and distribution of glycopyrrolate in the lungs with the high efficiency nebulizer provides the improved outcomes with nebulized glycopyrrolate that have heretofore eluded those skilled in the art employing conventional nebulizers for the administration of antimuscarinic agents such as glycopyrrolate.

### Example 3: Randomized, Cross-Over, Single Dose Study

At least about eight (8) adult healthy human volunteers (patients) are randomized to one of four treatment groups: (1) 100 µg aclidinium administered with a high efficiency nebulizer; (2) 300 µg of aclidinium administered with a high efficiency nebulizer; (3) 300 µg of aclidinium administered with a conventional nebulizer; (4) 900 µg of aclidinium administered with a conventional nebulizer. Blood samples are drawn immediately prior to administration of aclidinium and at predetermined time points thereafter. The blood plasma levels of aclidinium in the blood samples are determined and analyzed to determine the appropriate pharmacokinetic parameters (e.g. Cₘₐₓ, Tₘₐₓ, AUCₗₐₛₜ, and AUC_{0-∞}) for aclidinium. Additionally, the patients will be monitored for any adverse events and lung deposition (deposited lung dose) (measured by γ-scintigraphy), as well as vital signs and electrocardiogram.

A goal of this study is to verify that aclidinium administered to human patients with a high efficiency nebulizer at a relatively low dose produces in a patient or population of patients a pharmacokinetic profile characterized by a Cₘₐₓ, AUCₗₐₛₜ and/or AUC_{0-∞} that is comparable to, or greater than, a Cₘₐₓ, AUCₗₐₛₜ and/or AUC_{0-∞} obtained with a higher dose of aclidinium administered with a conventional nebulizer.

Another goal of this study is to verify that aclidinium administered to human patients with a high efficiency nebulizer produces in a patient or population of patients an improved adverse event profile as compared to a comparable or lower dose of aclidinium administered with a conventional nebulizer.

Another goal of this study is to verify that aclidinium administered to human patients with a high efficiency nebulizer produces in a patient or population of patients higher degree of lung deposition of the aclidinium as compared to a comparable or higher dose of aclidinium administered with a conventional nebulizer.

### Example 4: Randomized, Double-Blind, Placebo-Controlled Cross-Over, Single Dose Study

At least about twenty-four (24) adult human COPD patients of ages 45-65 years are randomized to one of three treatment groups: (1) 100 µg aclidinium administered with a high efficiency nebulizer; (2) 300 µg of aclidinium administered with a high efficiency nebulizer 900 µg of aclidinium administered with a high efficiency nebulized placebo.

Lung function is determined by spirometry, which measures e.g. FEV₁ and other suitable spirometry parameters. Spirometry is conducted immediately before and at predetermined intervals following administration of the aclidinium to the patients. Additionally, the patients will be monitored for any adverse events, as well as for vital signs and electrocardiogram.

A goal of this study is to verify that aclidinium administered to human patients with a high efficiency nebulizer at the tested doses produces in a patient or population of patients a therapeutic effect (i.e. at least one spirometry measurement, e.g. FEV₁, is at least 15% above baseline for a significant period of time, e.g. at least 4 hours, at least 6 hours, at least 8 hours, at least 12 hours, at least 18 hours or at least 24 hours.)

Another goal of this study is to verify that aclidinium administered to human patients with a high efficiency nebulizer produces in a patient or population of patients a suitable adverse event profile.

### Example 5: Randomized, Double-Blind, Placebo-Controlled, Parallel-Group, Multi-Dose Study

At least about forty-eight (48) adult human COPD patients of ages 45-65 years are randomized to one of four treatment groups: (1) 100 µg aclidinium administered B.I.D. with a high efficiency nebulizer; (2) 300 µg of aclidinium administered B.I.D. with a high efficiency nebulizer; (3) 300 µg of aclidinium administered Q.D. with a high efficiency nebulizer; (4) placebo administered B.I.D. with a high efficiency nebulizer.

Lung function is determined by spirometry, which measures e.g. FEV₁ and other suitable spirometry parameters. Spirometry is conducted immediately before and at predetermined intervals following administration of the aclidinium to the patients. Additionally, the patients will be monitored for any adverse events, as well as for vital signs and electrocardiogram.

COPD symptom scores are obtained by administering to each patient a conventional or proprietary symptom score instrument.

A goal of this study is to verify that aclidinium administered to human patients with a high efficiency nebulizer at the tested doses produces in a patient or population of patients a therapeutic effect (i.e. at least one spirometry measurement, e.g. FEV₁, is at least 15% above baseline for a significant period of time, e.g. at least 4 hours, at least 6 hours, at least 8 hours, at least 12 hours, at least 18 hour or at least 24 hours.)

Another goal of this study is to verify that aclidinium administered to human patients with a high efficiency nebulizer produces in a patient or population of patients a suitable adverse event profile.

### Example 6: Once-Per-Day (Q.D.) Dosing of Aclidinium

At least about three hundred (300) adult human COPD patients of ages >45 years are randomized to one of two treatment groups: (1) aclidinium administered with a high efficiency nebulizer; (2) placebo administered with a high efficiency nebulizer.

Lung function is determined by spirometry, which measures e.g. FEV₁ and other suitable spirometry parameters. Spirometry is conducted immediately before and at predetermined intervals following administration of the aclidinium to the patients. Additionally, the patients will be monitored for any adverse events, as well as for vital signs and electrocardiogram.

COPD symptom scores are obtained by administering to each patient a conventional or proprietary symptom score instrument.

A goal of this study is to verify that aclidinium administered to human patients with a high efficiency nebulizer at the tested doses produces in a patient or population of patients a therapeutic effect (i.e. at least one spirometry measurement, e.g. FEV₁, is at least 15% above baseline for a significant period of time, e.g. at least 4 hours, at least 6 hours, at least 8 hours, at least 12 hours, at least 18 hour or at least 24 hours.)

Another goal of this study is to verify that aclidinium administered to human patients with a high efficiency nebulizer produces in a patient or population of patients a suitable adverse event profile.

### Example 7: Randomized, Cross-Over, Single Dose Study

At least about eight (8) adult healthy human volunteers (patients) are randomized to one of four treatment groups: (1) 50 µg trospium administered with a high efficiency nebulizer; (2) 500 µg of trospium administered with a high efficiency nebulizer; (3) 500 µg of trospium administered with a conventional nebulizer; (4) 800 µg of trospium administered with a conventional nebulizer. Blood samples are drawn immediately prior to administration of trospium and at predetermined time points thereafter. The blood plasma levels of trospium in the blood samples are determined and analyzed to determine the appropriate pharmacokinetic parameters (e.g. Cₘₐₓ, Tₘₐₓ, AUCₗₐₛₜ, and AUC_{0-∞}) for trospium. Additionally, the patients will be monitored for any adverse events and lung deposition (measured by γ-scintigraphy), as well as vital signs and electrocardiogram.

A goal of this study is to verify that trospium administered to human patients with a high efficiency nebulizer at a relatively low dose produces in a patient or population of patients a pharmacokinetic profile characterized by a Cₘₐₓ, AUCₗₐₛₜ and/or AUC_{0-∞} that is comparable to, or greater than, a Cₘₐₓ, AUCₗₐₛₜ and/or AUC_{0-∞} obtained with a higher dose of trospium administered with a conventional nebulizer.

Another goal of this study is to verify that trospium administered to human patients with a high efficiency nebulizer produces in a patient or population of patients an improved adverse event profile as compared to a comparable dose of trospium administered with a conventional nebulizer.

Another goal of this study is to verify that trospium administered to human patients with a high efficiency nebulizer produces in a patient or population of patients higher degree of lung deposition of the trospium as compared to a comparable or higher dose of trospium administered with a conventional nebulizer.

### Example 8: Randomized, Double-Blind, Placebo-Controlled Cross-Over, Single Dose Study

At least about twenty-four (24) adult human COPD patients of ages 45-65 years are randomized to one of three treatment groups: (1) 50 µg trospium administered with a high efficiency nebulizer; (2) 500 µg of trospium administered with a high efficiency nebulizer; (3) placebo administered with a high efficiency nebulizer.

Lung function is determined by spirometry, which measures e.g. FEV₁ and other suitable spirometry parameters. Spirometry is conducted immediately before and at predetermined intervals following administration of the trospium to the patients. Additionally, the patients will be monitored for any adverse events, as well as for vital signs and electrocardiogram.

A goal of this study is to verify that trospium administered to human patients with a high efficiency nebulizer at the tested doses produces in a patient or population of patients a therapeutic effect (i.e. at least one spirometry measurement, e.g. FEV₁, is at least 15% above baseline for a significant period of time, e.g. at least 4 hours, at least 6 hours, at least 8 hours, at least 12 hours, at least 18 hour or at least 24 hours.)

Another goal of this study is to verify that trospium administered to human patients with a high efficiency nebulizer produces in a patient or population of patients a suitable adverse event profile.

### Example 9: Randomized, Double-Blind, Placebo-Controlled, Parallel-Group, Multi-Dose Study

At least about forty-eight (48) adult human COPD patients of ages 45-65 years are randomized to one of four treatment groups: (1) 50 µg trospium administered B.I.D. with a high efficiency nebulizer; (2) 500 µg of trospium administered B.I.D. with a high efficiency nebulizer; (3) 500 µg of trospium administered Q.D. with a high efficiency nebulizer; (4) placebo administered B.I.D. with a high efficiency nebulizer.

Lung function is determined by spirometry, which measures e.g. FEV₁ and other suitable spirometry parameters. Spirometry is conducted immediately before and at predetermined intervals following administration of the trospium to the patients. Additionally, the patients will be monitored for any adverse events, as well as for vital signs and electrocardiogram.

COPD symptom scores are obtained by administering to each patient a conventional or proprietary symptom score instrument.

A goal of this study is to verify that trospium administered to human patients with a high efficiency nebulizer at the tested doses produces in a patient or population of patients a therapeutic effect (i.e. at least one spirometry measurement, e.g. FEV₁, is at least 15% above baseline for a significant period of time, e.g. at least 4 hours, at least 6 hours, at least 8 hours, at least 12 hours, at least 18 hour or at least 24 hours.)

Another goal of this study is to verify that trospium administered to human patients with a high efficiency nebulizer produces in a patient or population of patients a suitable adverse event profile.

### Example 10: Twice-Per-Day (B.I.D.) Dosing of Trospium

At least about three hundred (300) adult human COPD patients of ages >45 years are randomized to one of two treatment groups: (1) trospium administered with a high efficiency nebulizer; (2) placebo administered with a high efficiency nebulizer.

Lung function is determined by spirometry, which measures e.g. FEV₁ and other suitable spirometry parameters. Spirometry is conducted immediately before and at predetermined intervals following administration of the trospium to the patients. Additionally, the patients will be monitored for any adverse events, as well as for vital signs and electrocardiogram.

COPD symptom scores are obtained by administering to each patient a conventional or proprietary symptom score instrument.

A goal of this study is to verify that trospium administered to human patients with a high efficiency nebulizer at the tested doses produces in a patient or population of patients a therapeutic effect (i.e. at least one spirometry measurement, e.g. FEV₁, is at least 15% above baseline for a significant period of time, e.g. at least 4 hours, at least 6 hours, at least 8 hours, at least 12 hours, at least 18 hour or at least 24 hours.)

Another goal of this study is to verify that trospium administered to human patients with a high efficiency nebulizer produces in a patient or population of patients a suitable adverse event profile.

### Example 11: Randomized, Cross-Over, Single Dose Study

At least about eight (8) adult healthy human volunteers (patients) are randomized to one of five treatment groups: (1) 0.5 mg glycopyrrolate administered with a high efficiency nebulizer; (2) 1.0 mg of glycopyrrolate administered with a high efficiency nebulizer; (3) 1.0 mg of glycopyrrolate administered with a conventional nebulizer; (4) 2.0 mg of glycopyrrolate administered with a conventional nebulizer; (5) 0.25 mg of glycopyrrolate administered intravenously. Blood samples are drawn immediately prior to administration of glycopyrrolate and at predetermined time points thereafter. The blood plasma levels of glycopyrrolate in the blood samples are determined and analyzed to determine the appropriate pharmacokinetic parameters (e.g. Cₘₐₓ, Tₘₐₓ, AUCₗₐₛₜ, and AUC_{0-∞}) for glycopyrrolate. Additionally, the patients will be monitored for any adverse events and lung deposition (measured by γ-scintigraphy), as well as vital signs and electrocardiogram.

A goal of this study is to verify that glycopyrrolate administered to human patients with a high efficiency nebulizer at a relatively low dose produces in a patient or population of patients a pharmacokinetic profile characterized by a Cₘₐₓ, AUCₗₐₛₜ and/or AUC_{0-∞} that is comparable to, or greater than, a Cₘₐₓ, AUCₗₐₛₜ and/or AUC_{0-∞} obtained with a higher dose of glycopyrrolate administered with a conventional nebulizer.

Another goal of this study is to verify that glycopyrrolate administered to human patients with a high efficiency nebulizer produces in a patient or population of patients an improved adverse event profile as compared to a comparable dose of glycopyrrolate administered with a conventional nebulizer.

Another goal of this study is to verify that glycopyrrolate administered to human patients with a high efficiency nebulizer produces in a patient or population of patients higher degree of lung deposition of the glycopyrrolate as compared to a comparable or higher dose of glycopyrrolate administered with a conventional nebulizer.

### Example 12: Randomized, Double-Blind, Placebo-Controlled Cross-Over, Single Dose Study

At least about twenty-four (24) adult human COPD patients of ages 45-65 years are randomized to one of three treatment groups: (1) 0.5 mg glycopyrrolate administered with a high efficiency nebulizer; (2) 1.0 mg of glycopyrrolate administered with a high efficiency nebulizer; (3) placebo administered with a high efficiency nebulizer.

Lung function is determined by spirometry, which measures e.g. FEV₁ and other suitable spirometry parameters. Spirometry is conducted immediately before and at predetermined intervals following administration of the glycopyrrolate to the patients. Additionally, the patients will be monitored for any adverse events, as well as for vital signs and electrocardiogram.

A goal of this study is to verify that glycopyrrolate administered to human patients with a high efficiency nebulizer at the tested doses produces in a patient or population of patients a therapeutic effect (i.e. at least one spirometry measurement, e.g. FEV₁, is at least 15% above baseline for a significant period of time, e.g. at least 4 hours, at least 6 hours, at least 8 hours, at least 12 hours, at least 18 hour or at least 24 hours.)

Another goal of this study is to verify that glycopyrrolate administered to human patients with a high efficiency nebulizer produces in a patient or population of patients a suitable adverse event profile.

### Example 13: Randomized, Double-Blind, Placebo-Controlled, Parallel-Group, Multi-Dose Study

At least about forty-eight (48) adult human COPD patients of ages 45-65 years are randomized to one of four treatment groups: (1) 0.5 mg glycopyrrolate administered B.I.D. with a high efficiency nebulizer; (2) 1.0 mg of glycopyrrolate administered B.I.D. with a high efficiency nebulizer; (3) 1.0 mg of glycopyrrolate administered Q.D. with a high efficiency nebulizer; (4) placebo administered B.I.D. with a high efficiency nebulizer.

Lung function is determined by spirometry, which measures e.g. FEV₁ and other suitable spirometry parameters. Spirometry is conducted immediately before and at predetermined intervals following administration of the glycopyrrolate to the patients. Additionally, the patients will be monitored for any adverse events, as well as for vital signs and electrocardiogram.

COPD symptom scores are obtained by administering to each patient a conventional or proprietary symptom score instrument.

A goal of this study is to verify that glycopyrrolate administered to human patients with a high efficiency nebulizer at the tested doses produces in a patient or population of patients a therapeutic effect (i.e. at least one spirometry measurement, e.g. FEV₁, is at least 15% above baseline for a significant period of time, e.g. at least 4 hours, at least 6 hours, at least 8 hours, at least 12 hours, at least 18 hour or at least 24 hours.)

Another goal of this study is to verify that glycopyrrolate administered to human patients with a high efficiency nebulizer produces in a patient or population of patients a suitable adverse event profile.

### Example 14: Once-Per-Day (Q.D.) Dosing of Glycopyrrolate

At least about three hundred (300) adult human COPD patients of ages >45 years are randomized to one of two treatment groups: (1) glycopyrrolate (1.0 or 0.5 mg/day) administered with a high efficiency nebulizer; (2) placebo administered with a high efficiency nebulizer.

Lung function is determined by spirometry, which measures e.g. FEV₁ and other suitable spirometry parameters. Spirometry is conducted immediately before and at predetermined intervals following administration of the glycopyrrolate to the patients. Additionally, the patients will be monitored for any adverse events, as well as for vital signs and electrocardiogram.

COPD symptom scores are obtained by administering to each patient a conventional or proprietary symptom score instrument.

A goal of this study is to verify that glycopyrrolate administered to human patients with a high efficiency nebulizer at the tested doses produces in a patient or population of patients a therapeutic effect (i.e. at least one spirometry measurement, e.g. FEV1, is at least 15% above baseline for a significant period of time, e.g. at least 4 hours, at least 6 hours, at least 8 hours, at least 12 hours, at least 18 hour or at least 24 hours.)

Another goal of this study is to verify that glycopyrrolate administered to human patients with a high efficiency nebulizer produces in a patient or population of patients a suitable adverse event profile.

### Example 15: Aerosol Characterization of Glycopyrrolate Solution for Inhalation (2 mg/mL)

### Objective

The object of the study was to determine the drug delivery efficiency of three different nebulizer systems, PARI eFlow® high efficiency nebulizer, LC® PLUS conventional nebulizer and Hudson RCI MicroMist® conventional nebulizer, using a glycopyrrolate inhalation solution (GIS). Droplet size and respirable fraction of the aerosol were measured by laser diffraction, while delivered dose, nebulization time and nebulizer residue were assessed by breath simulation using a standard adult breathing pattern.

### Summary

Three different nebulizers were characterized by laser diffraction and breath simulation. Jet nebulizers (PARI LC® PLUS and MicroMist® were operated with 3 PARI Boy N compressors; eFlow® 30 L were combined with 3 eFlow® rapid control units. Consequently, 18 laser diffraction and 18 breath simulation experiments in total were carried out. The parameters DD, nebulization time, MMD, GSD, RD < 5 µm and DDR were determined upon these measurements.

The PARI eFlow® nebulizer generated droplets of a mass median diameter (MMD) of around 3.5 µm and a geometric standard deviation (GSD) of 1.55 compared to the MMD and GSD obtained from jet nebulizers PARI LC® PLUS (3.8 µm, 2.32) and MicroMist® (4.6 µm, 1.99).

To evaluate the delivered dose breath simulator experiments were performed with adult breathing pattern. For the eFlow® 30 L the delivered dose was 667 µg; for PARI LC® PLUS 985 µg was obtained. A delivered dose of 629 µg using the MicroMist® was determined. The nebulization time for the jet nebulizers LC® PLUS and Hudson RCI MicroMist® was about 3.4 min. and 3.7 min., respectively. The eFlow® 30 L required 1.4 min.

The respirable dose is calculated by multiplying the delivered dose, obtained in breath simulation experiments, with the respirable fractions determined by laser diffraction. The PARI eFlow® 30 L had a respirable dose < 5 µm (RD) of 532 µg compared to the RD < 5 µm obtained from the jet nebulizers PARI LC® PLUS and MicroMist®.

### Materials and Methods

The drug formulation was a 2 mg/mL glycopyrrolate solution (GIS). This solution was tested at the following quantities (mg) and volumes (mL): PARI eFlow® 30 L : 1mg/0.5 mL; PARI LC® PLUS and MicroMist®: 4 mg/2 mL.

Breath simulation tests were conducted according to EP Section 2.9.44 and proprietary test methods using the PARI breath simulator with adult breathing pattern (500 mL tidal volumes, 15 breaths/min, inhalation/exhalation ratio 50:50).

The nebulizers were equipped with expiratory filters and connected via an inspiratory filter to the breathing simulator. According to EP Section 2.9.44 the inspiratory filter was changed after the first minute, the second filter remained until the end of nebulization. The end of the nebulization was reached for the PARI eFlow® 30 L when the nebulizer switched off automatically; for the jet nebulizers, the end of nebulization was achieved one minute after sputtering begins.

The parameters measured for breath simulation are delivered dose (DD), drug in residue and nebulization time.

Laser diffraction was used to assess the geometric droplet size distribution with a Malvern MasterSizerX® laser diffraction measurement device. Airflow was 15 L/min ± 0.1 L/min; temperature was 23°C ± 2°C; relative humidity was 50% ± 5%. The parameters obtained with laser diffraction were: mass median diameter of the droplets (MMD), respirable fraction (RF), geometric standard deviation (GSD), and total output rate (TOR).

The calculated parameters were respirable dose (RD), drug delivery rate (DDR) and respirable drug delivery rate (RDDR).

Breath simulation experiment results are set forth in Table 15-1. Laser diffraction measurements are set forth in Table 15-2. Results of *in vitro* experiments with the PARI eFlow® 30 L, PARI LC® PLUS and Aeroneb® Go are set forth in Table 15-3.

**Table 15-1: Breath Simulation Experiments: Mean breath simulation results upon nebulization of 4 mg / 2 ml utilizing jet nebulizers and 1 mg / 0.5 ml using the eFlow 30L**

| | | Delivered Dose | | Drug in Residue | | Aerosol losses | | Neb. Time |
|---|---|---|---|---|---|---|---|---|
| | | µg | % | µg | % | µg | % | min |
| LC® PLUS + PARI BOY N | mean | 985 | 24.4 | 2513 | 62.4 | 532 | 13.2 | 3.40 |
| | SD | 85 | 2.1 | 97 | 2.3 | 59 | 1.5 | 0.32 |
| | RSD | 8.6 | 8.7 | 3.8 | 3.7 | 11.1 | 11.2 | 9.5 |
| Hudson MicroMist® + PARI BOY® N | mean | 629 | 15.6 | 2843 | 70.3 | 570 | 14.1 | 3.65 |
| | SD | 37 | 0.9 | 142 | 3.5 | 119 | 2.9 | 0.3 |
| | RSD | 5.9 | 5.8 | 5.0 | 5.0 | 20.9 | 20.9 | 8.0 |
| eFlow® 30L | mean | 667 | 65.9 | 113 | 11.2 | 232 | 22.9 | 1.36 |
| | SD | 28 | 2.8 | 27 | 2.7 | 25 | 2.4 | 0.09 |
| | RSD | 4.2 | 4.2 | 24.1 | 24.3 | 10.6 | 10.4 | 7.0 |

**Table 15-2: Laser Diffraction Measurements: Mean laser diffraction and calculated results upon nebulization of 4 mg / 2 ml utilizing jet nebulizers and 1 mg / 0.5 ml using the eFlow® 30L**

| | | LC® PLUS + PARI BOY® N | | | Hudson MicroMist + PARI BOY® N | | | eFlow® 30L | | |
|---|---|---|---|---|---|---|---|---|---|---|
| | | mean | SD | RSD | mean | SD | RSD | mean | SD | RSD |
| MMD | µm | 3.82 | 0.31 | 8.2 | 4.55 | 0.37 | 8.2 | 3.46 | 0.13 | 3.8 |
| GSD | | 2.32 | 0.13 | 5.6 | 1.99 | 0.04 | 2.2 | 1.55 | 0.03 | 1.9 |
| TOR | mg/min | 382 | 48 | 12.5 | 224 | 14 | 6.2 | 631 | 121 | 19.2 |
| Respirable Fraction <5µm | % | 63.9 | 4.0 | 6.3 | 56.0 | 5.2 | 9.3 | 79.7 | 3.3 | 4.1 |
| Respirable Fraction <3.3µm | % | 42.7 | 4.2 | 9.8 | 32.6 | 4.2 | 12.9 | 46.0 | 3.2 | 7.0 |
| Respirable Fraction <2µm | % | 22.4 | 3.0 | 13.3 | 14.8 | 2.0 | 13.7 | 10.6 | 0.5 | 4.5 |
| Respirable Dose <5µm | µg | 630 | 79 | 12.5 | 352 | 40 | 11.3 | 532 | 43 | 8.1 |
| | % | 15.6 | 2.0 | 12.6 | 8.7 | 1.0 | 11.3 | 52.6 | 4.3 | 8.2 |
| Respirable Dose <3.3µm | µg | 422 | 64 | 15.1 | 205 | 31 | 15.0 | 307 | 33 | 10.8 |
| | % | 10.5 | 1.6 | 15.2 | 5.10 | 0.80 | 15.0 | 30.4 | 3.3 | 11.0 |
| Respirable Dose <2µm | µg | 221 | 40 | 18.0 | 94 | 15 | 15.9 | 71 | 5 | 6.9 |
| | % | 5.5 | 1.0 | 18.1 | 2.3 | 0.4 | 15.9 | 7.0 | 0.5 | 7.0 |
| DDR | µg/min | 338 | 26 | 7.8 | 265 | 31 | 11.7 | 494 | 41.0 | 8.2 |
| | %/min | 8.4 | 0.7 | 7.7 | 6.6 | 0.8 | 11.8 | 48.8 | 4.1 | 8.5 |
| RDDR <5µm | µg/min | 215 | 9 | 4.3 | 149 | 25 | 16.7 | 394 | 47 | 12.0 |
| | %/min | 5.3 | 0.2 | 4.2 | 3.7 | 0.6 | 16.8 | 39.0 | 4.8 | 12.2 |
| RDDR <3.3µm | µg/min | 144 | 9 | 6.3 | 87 | 17 | 20.1 | 228 | 34 | 14.8 |
| | %/min | 3.6 | 0.2 | 6.2 | 2.2 | 0.4 | 20.1 | 22.5 | 3.4 | 0.5 |
| RDDR <2µm | µg/min | 75 | 7 | 9.3 | 40 | 8 | 20.8 | 53 | 5 | 10.3 |
| | %/min | 1.9 | 0.2 | 9.2 | 1.0 | 0.2 | 20.9 | 5.2 | 0.5 | 10.5 |

**Table 15-3: Comparison of In Vitro Results obtained with Different Nebulizers**

| **Nebulizer** | **eFlow® 30L** | **LC® Plus** |
|---|---|---|
| Laser Diffraction | | |
| MMD [µm] | 3.5 | 3.8 |
| GSD | 1.6 | 2.3 |
| Fine Particle Fraction [%<5µ] | 80% | 64% |
| Breath Simulation | | |
| Inhalation filter [%] | 66% | 24% |
| Efficiency | | |
| **Respirable dose [%]** | **53%** | **16%** |

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

The following Embodiments 1 to 77 are embodiments of the invention:
1. A method of treating a patient having chronic obstructive pulmonary disease (COPD), comprising administering to the patient, with a high efficiency nebulizer, a nominal dose of a composition comprising a muscarinic antagonist that provides the patient with a therapeutic effect for at least about 24 hours.
2. The method of Embodiment 1, wherein administering said nominal dose with the high efficiency nebulizer produces in the patient therapeutically acceptable side effects.
3. The method of Embodiment 1, wherein administering said nominal dose of the composition with the high efficiency nebulizer provides to the patient reduced side effects compared to administering the same nominal dose with a conventional nebulizer.
4. The method of Embodiment 1, wherein administering said nominal dose of the composition with the high efficiency nebulizer produces a calculated respirable dose of the muscarinic antagonist, whereby the patient experiences reduced side effects compared to administering a nominal dose that is calculated to achieve the same respirable dose with a conventional nebulizer.
5. The method of Embodiment 1, wherein administering said nominal dose of the composition with the high efficiency nebulizer achieves a deposited lung dose of the muscarinic antagonist, whereby the patient experiences reduced side effects compared to administering a nominal dose that achieves substantially the same deposited lung dose with a conventional nebulizer.
6. The method of Embodiment 1, wherein the composition comprising the muscarinic antagonist is a concentrated, preservative-free, pH-adjusted solution formulation of the muscarinic antagonist.
7. The method of Embodiment 6, wherein the concentration of the muscarinic antagonist is greater than about 0.25 mg/mL.
8. The method of Embodiment 6, wherein the composition has a pH of 3 to 5.
9. The method of Embodiment 6, wherein the composition is stable at room temperature for more than two years.
10. The method of Embodiment 6, wherein the composition comprising the muscarinic antagonist contains about 50 µg to about 1000 µg of glycopyrrolate as the muscarinic antagonist.
11. The method of Embodiment 1, wherein the composition has a volume of about 0.5 mL or less.
12. The method of Embodiment 1, wherein the composition is administered in about 3 minutes or less.
13. The method of Embodiment 1, wherein the solution has a stabilizing excipient.
14. The method of Embodiment 13, wherein the stabilizing excipient is ethylenediaminetetraacetic acid (EDTA) or a pharmaceutically acceptable salt thereof.
15. The method of Embodiment 1, wherein the composition further comprises an excipient to mitigate side effects, for example dry mouth.
16. The method of Embodiment 15, wherein the excipient comprises citric acid or a pharmaceutically acceptable salt thereof.
17. The method of Embodiment 1, wherein the muscarinic antagonist is a long-acting muscarinic antagonist.
18. The method of Embodiment 1, wherein the nominal dose of the composition comprising the muscarinic antagonist contains about 50 µg to about 1000 µg of glycopyrrolate as the muscarinic antagonist.
19. The method of Embodiment 1, wherein the nominal dose of the composition comprising the muscarinic antagonist contains about 50 µg to about 500 µg of glycopyrrolate as the muscarinic antagonist.
20. The method of Embodiment 19, wherein the composition comprising the muscarinic antagonist contains about 50 µg to about 300 µg of glycopyrrolate as the muscarinic antagonist.
21. The method of Embodiment 20, wherein the composition comprising the muscarinic antagonist contains about 50 µg to about 150 µg of glycopyrrolate as the muscarinic antagonist.
22. The method of Embodiment 1, wherein the high efficiency nebulizer emits droplets having a Mass Median Aerodynamic Diameter (MMAD) of less than about 4.5 µm and a geometric standard deviation (GSD) of less than about 2.0, an MMAD of less than 4.0 µm and a GSD less than 1.8, or optimally an MMAD less than 3.6 and a GSD less than 1.6.
23. The method of Embodiment 1, wherein the therapeutic effect comprises an improvement of FEV₁ above baseline of at least about 10% at 24 hours after the composition is administered with the high efficiency nebulizer.
24. The method of Embodiment 1, wherein the therapeutic effect comprises an improvement of FEV₁ above baseline of at least about 100 mL at 24 hours after the composition is administered with the high efficiency nebulizer.
25. The method of Embodiment 1, wherein the composition further comprises a beta 2-adrenoreceptor agonist, a corticosteroid, or both.
26. A method of treating a patient having chronic obstructive pulmonary disease (COPD), comprising administering to the patient, with a high efficiency nebulizer, a nominal dose of a composition comprising a muscarinic antagonist, wherein administering said nominal dose with said high efficiency nebulizer provides to the patient: (1) an increased magnitude and/or duration of therapeutic effect; and (2) reduced or acceptable side effects, compared to administering the same nominal dose of the muscarinic antagonist with a conventional nebulizer.
27. The method of Embodiment 26, wherein administering said nominal dose with the high efficiency nebulizer produces in the patient therapeutically acceptable side effects.
28. The method of Embodiment 26, wherein administering said nominal dose of the composition with the high efficiency nebulizer provides to the patient reduced side effects compared to administering the same nominal dose with a conventional nebulizer.
29. The method of Embodiment 26, wherein administering said nominal dose of the composition with the high efficiency nebulizer produces a calculated respirable dose of the muscarinic antagonist, whereby the patient experiences reduced side effects compared to administering a nominal dose that is calculated to achieve the same respirable dose with a conventional nebulizer.
30. The method of Embodiment 26, wherein administering said nominal dose of the composition with the high efficiency nebulizer achieves a deposited lung dose of the muscarinic antagonist, whereby the patient experiences reduced side effects compared to administering a nominal dose that achieves substantially the same deposited lung dose with a conventional nebulizer.
31. The method of Embodiment 26, wherein the composition comprising the muscarinic antagonist is a concentrated, preservative-free, pH-adjusted solution formulation of the muscarinic antagonist.
32. The method of Embodiment 31, wherein the concentration of the muscarinic antagonist is greater than about 0.25 mg/mL.
33. The method of Embodiment 31, wherein the composition has a pH of 3 to 5.
34. The method of Embodiment 31, wherein the composition comprising the muscarinic antagonist contains about 50 µg to about 1000 µg of glycopyrrolate as the muscarinic antagonist.
35. The method of Embodiment 26, wherein the composition has a volume of about 0.5 mL or less.
36. The method of Embodiment 26, wherein the composition is administered in about 3 minutes or less.
37. The method of Embodiment 26, wherein the solution has a stabilizing excipient.
38. The method of Embodiment 37, wherein the stabilizing excipient is ethylenediaminetetraacetic acid (EDTA) or a pharmaceutically acceptable salt thereof.
39. The method of Embodiment 26, wherein the composition further comprises an excipient to mitigate side effects, for example dry mouth.
40. The method of Embodiment 39, wherein the excipient comprises citric acid or a pharmaceutically acceptable salt thereof.
41. The method of Embodiment 26, wherein the muscarinic antagonist is a long-acting muscarinic antagonist.
42. The method of Embodiment 26, wherein the high efficiency nebulizer emits droplets having a Mass Median Aerodynamic Diameter (MMAD) of less than about 4.5 µm and a geometric standard deviation (GSD) of less than about 2.0, an MMAD of less than 4.0 µm and a GSD less than 1.8, or optimally an MMAD less than 3.6 and a GSD less than 1.6.
43. The method of Embodiment 26, wherein the therapeutic effect comprises an improvement of FEV₁ above baseline of at least about 10% at 24 hours after the composition is administered with the high efficiency nebulizer.
44. The method of Embodiment 26, wherein the therapeutic effect comprises an improvement of FEV₁ above baseline of at least about 100 mL at 24 hours after the composition is administered with the high efficiency nebulizer
45. The method of Embodiment 26, wherein the composition further comprises a beta 2-adrenoreceptor agonist, a corticosteroid, or both.
46. A method of treating a patient having chronic obstructive pulmonary disease (COPD), comprising administering to the patient, with a high efficiency nebulizer, a nominal dose calculated to produce a respirable dose of a composition comprising a muscarinic antagonist, wherein producing said calculated respirable dose with said high efficiency nebulizer provides to the patient: (1) at least similar magnitude and/or duration of therapeutic effect; and (2) reduced side effects, compared to administering a nominal dose calculated to produce substantially the same respirable dose of the muscarinic antagonist with a conventional nebulizer.
47. The method of Embodiment 46, wherein the composition comprising the muscarinic antagonist is a concentrated, preservative-free, pH-adjusted solution formulation of the muscarinic antagonist.
48. The method of Embodiment 47, wherein the concentration of the muscarinic antagonist is greater than about 0.25 mg/mL.
49. The method of Embodiment 46, wherein the solution has a stabilizing excipient.
50. The method of Embodiment 46, wherein the composition further comprises an excipient to mitigate side effects, for example dry mouth.
51. A method of treating a patient having chronic obstructive pulmonary disease (COPD), comprising administering to the patient, with a high efficiency nebulizer, a nominal dose that achieves a deposited lung dose of a composition comprising a muscarinic antagonist, wherein producing said deposited lung dose with said high efficiency nebulizer provides to the patient: (1) at least similar magnitude and/or duration of therapeutic effect; and (2) reduced side effects, compared to administering a nominal dose that achieves substantially the same deposited lung dose of the composition comprising the muscarinic antagonist with a conventional nebulizer.
52. The method of Embodiment 51, wherein the composition comprising the muscarinic antagonist is a concentrated, preservative-free, pH-adjusted solution formulation of the muscarinic antagonist.
53. The method of Embodiment 52, wherein the concentration of the muscarinic antagonist is greater than about 0.25 mg/mL.
54. The method of Embodiment 51, wherein the solution has a stabilizing excipient.
55. The method of Embodiment 51, wherein the composition further comprises an excipient to mitigate side effects, for example dry mouth.
56. A method of treating a patient having chronic obstructive pulmonary disease (COPD), comprising administering to the patient, with a high efficiency nebulizer, a nominal dose of a composition containing less than about 100 µg of glycopyrrolate, whereby said patient experiences a therapeutic effect, with therapeutically acceptable side effects, for at least about 12 hours.
57. The method of Embodiment 56, wherein said patient experiences a therapeutic effect for at least about 18 hours.
58. The method of Embodiment 56, wherein said patient experiences a therapeutic effect for at least about 24 hours.
59. The method of Embodiment 56, wherein the composition comprising the muscarinic antagonist is a concentrated, preservative-free, pH-adjusted solution formulation of the muscarinic antagonist.
60. A method of treating a patient having chronic obstructive pulmonary disease (COPD), comprising administering to the patient, with a nebulizer, a nominal dose of a composition comprising a muscarinic antagonist that is effective to provide the patient with a therapeutic effect for at least about 24 hours.
61. The method of Embodiment 60, wherein administering said nominal dose with the nebulizer produces in the patient therapeutically acceptable side effects.
62. The method of Embodiment 60, wherein the composition comprising the muscarinic antagonist is a concentrated, preservative-free, pH-adjusted solution formulation of the muscarinic antagonist.
63. A composition for administration with a high efficiency nebulizer, comprising a concentrated, preservative-free, pH-adjusted solution formulation of the muscarinic antagonist.
64. The composition of Embodiment 63, wherein the muscarinic antagonist is glycopyrrolate.
65. The composition of Embodiment 64, wherein the glycopyrrolate has a concentration of at least 0.25 mg/mL.
66. The composition of Embodiment 63, wherein the pH is about 3 to about 5.
67. The composition of Embodiment 63, wherein the composition further comprises a beta 2-adrenoreceptor agonist, a corticosteroid, or both.
68. The composition of Embodiment 63, wherein the composition is stable at room temperature for more than two years.
69. The use of a muscarinic antagonist in the manufacture of a medicament for the treatment of chronic obstructive pulmonary disease (COPD) wherein the medicament is administered from a high efficiency nebulizer.
70. The use of Embodiment 69, wherein the muscarinic antagonist is glycopyrrolate.
71. The use of Embodiment 70, wherein the glycopyrrolate has a concentration of at least 0.25 mg/mL.
72. The use of Embodiment 69, wherein the pH is about 3 to about 5.
73. A medicament comprising a muscarinic antagonist for the treatment of COPD by administration from a high efficiency nebulizer.
74. The medicament of Embodiment 73, wherein the muscarinic antagonist is glycopyrrolate.
75. The medicament of Embodiment 74, wherein the glycopyrrolate has a concentration of at least 0.25 mg/mL.
76. The medicament of Embodiment 73, wherein the pH is about 3 to about 5.
77. The medicament of Embodiment 73, wherein the composition is stable at room temperature for more than two years.

## Claims

1. A composition for use in a method of treating a patient having chronic obstructive pulmonary disease (COPD), wherein the composition contains glycopyrrolate in an amount of less than 100 µg/dose, and wherein the composition is administered to the patient using a high efficiency nebulizer that emits droplets of the composition having a Mass Mean Aerodynamic Diameter (MMAD) as measured by laser diffraction of less than 4.5 µm and a geometric standard deviation (GSD) of less than 2.0, and wherein said method comprises administering a dose of said composition to a patient in order to treat said patient for at least 12 hours.

2. The composition for use according to claim 1, wherein the MMAD is less than 4.0 µm and the GSD is less than 1.8.

3. The composition for use according to claim 1 or 2, wherein the MMAD is less than 3.6 µm and the GSD is less than 1.6.

4. The composition for use according to any preceding claim, comprising equal to or less than 80 µg/dose.

5. The composition for use according to any preceding claim, wherein the high efficiency nebulizer provides a respirable fraction (RF) of glycopyrrolate of least 60%, at least 65%, at least 70%, at least 75%, at least 80%, at least 85%, at least 90%, 60% to 95%, 65% to 95%, 65% to 90% or 70% to 90%.

6. The composition for use according to any preceding claim, whereby said patient experiences a therapeutic effect, with therapeutically acceptable side effects, for at least 12 hours.

7. The composition for use according to any preceding claim, whereby said patient experiences a therapeutic effect, with therapeutically acceptable side effects, for at least 18 hours.

8. The composition for use according to any preceding claim, whereby said patient experiences a therapeutic effect, with therapeutically acceptable side effects, for at least 24 hours.
